# FASCICULE DE BREVET EUROPEEN

(11) **EP 3 052 500 B1**
(45) Date de publication et mention de la délivrance du brevet: **03.01.2018**
(21) Numéro de dépôt: 14796208.8
(22) Date de dépôt: 29.09.2014
(51) Int. Cl.: A61K 45/06, A61K 31/675, A61K 31/7076, A61K 31/706, A61K 31/7064, A61K 31/7068, A61K 31/7072, A61K 31/708, C07F 9/6561

(54) **INHIBITEURS DE 5'-NUCLÉOTIDASES ET LEURS UTILISATIONS THÉRAPEUTIQUES**
5'-NUCLEOTIDASE-HEMMER UND DEREN THERAPEUTISCHE VERWENDUNGEN
INHIBITORS OF 5'-NUCLEOTIDASES AND THERAPEUTIC USES THEREOF

(30) Priorité: 01.10.2013 FR 1359472
(43) Date de publication de la demande: 10.08.2016
(73) Titulaire: Centre National de la Recherche Scientifique, 75794 Paris Cedex 16 (FR); Les Hospices Civils de Lyon, 69002 Lyon (FR); Ecole Normale Supérieure de Lyon, 69342 Lyon Cedex 07 (FR); Université Claude Bernard Lyon 1, 69922 Villeurbanne (FR); Université de Montpellier 1, 34067 Montpellier Cedex 2 (FR)
(72) Inventeur: CHALOIN, Laurent, F-34730 Prades Le Lez (FR); PEYROTTES, Suzanne, F-34790 Grabels (FR); LIONNE, Corinne, F-34090 Montpellier (FR); MARTON, Zsuzsanna, F-44500 La Baule-escoublac (FR); EGRON, David, F-34790 Grabels (FR); GUILLON, Rémi, 34570 Montarnaud (FR); PERIGAUD, Christian, F-34790 Grabels (FR); DUMONTET, Charles, F-69200 Venissieux (FR); JORDHEIM, Lars Petter, F-69800 Saint-priest (FR); KRIMM, Isabelle, F-69730 Genay (FR)
(74) Mandataire: Cabinet Plasseraud
(86) Numéro de dépôt international: PCT/FR2014/052447
(87) Numéro de publication internationale: WO 2015/049447

(56) Documents cités:
- WO-A1-00/44750
- WO-A1-98/05335
- WALTER RIED ET AL.: "Strukturaufklärung von N6-, 9- und 7-Acyladeninen durch 1H- und 13C-NMR-Spektroskopie von Festförpern und in Lösung", HELVETICA CHIMICA ACTA., vol. 72, 1989, pages 1597-1607, XP002725623, CHVERLAG HELVETICA CHIMICA ACTA, BASEL. ISSN: 0018-019X

## Description

La présente invention concerne de nouveaux composés possédant un squelette de type 6-amino-purine comme inhibiteurs de 5'-nucléotidases, et notamment de la 5'-nucléotidase cytosolique II (cN-II). L'invention concerne également l'utilisation desdits composés, seuls ou en association avec des analogues de nucléosides et/ou de nucléobases, en chimiothérapie anti-cancéreuse.

Les analogues de nucléosides représentent une classe d'agents thérapeutiques largement utilisée dans le traitement des hémopathies malignes et des tumeurs solides. Leur mode d'action repose sur une métabolisation intracellulaire en leurs dérivés phosphorylés (nucléotides) qui interfèrent avec les systèmes enzymatiques impliqués dans la biosynthèse des acides nucléiques (polymérases, ribonucléotide réductase, thymidilate synthétase ...).

Les analogues de nucléobases tels que les dérivés du type 6-mercapto-purines ont à ce jour été décrits pour le traitement des leucémies aiguës lymphoblastiques, les leucémies aiguës myéloblastiques, et les leucémies myéloïdes chroniques chez l'adulte et l'enfant¹. Les médicaments anticancéreux administrés par voie orale en pédiatrie sont peu nombreux, mais fréquemment utilisés dans divers cas de pathologies malignes tels que les leucémies, la maladie de Hodgkin, les lymphomes non hodgkiniens, les tumeurs cérébrales. Leur mode d'action est similaire aux analogues de nucléosides.

L'utilisation d'analogues de nucléosides se heurte néanmoins à l'apparition de phénomènes de résistance, impliquant notamment une dérégulation enzymatique entre l'expression intracellulaire de kinases et de 5'-nucléotidases. Ainsi, l'expression élevée de l'activité de 5'-nucléotisases au diagnostic est connue pour être un facteur prédictif indépendant défavorable de survie chez des patients atteints de cancers. A titre d'exemple, l'expression élevée de la 5'-nucléotidase cytosolique II (cN-II) est observée cliniquement chez des patients atteints de leucémie aiguë myéloïde ayant une survie globale moins bonne que les autres patients. De plus, de récents travaux^{2a,b} ont montré l'existence de mutations stimulant l'activité de la cN-II humaine entraînant une résistance accrue aux traitements par la 6-mercaptopurine et la 6-thioguanine utilisées en pédiatrie.

Aucune réponse thérapeutique n'a cependant été apportée à ce jour à la modulation des concentrations nucléotidiques résultant de l'expression ou de la surexpression de 5'-nucléotidases.

Le document US 2010/0204182 décrit des inhibiteurs d'ectonucléotidases pour le traitement de maladies diverses et variées telles que la sécheresse oculaire, les maladies respiratoires, la fibrose kystique, les maladies inflammatoires, les maladies du système immunitaire, les troubles gastro-intestinaux, les troubles rénaux, les cancers et les maladies du cerveau.

Cependant, la cible pharmacologique dans ce document est membranaire (et non cytosolique) et les applications visées sont extrêmement larges.

Le document « Biochem. J. (1989) 262, 203-8 » décrit des inhibiteurs de 5'-nucléotidases cytosoliques présentes dans des extraits de foie et de coeur de rats; ces composés appartiennent à la famille des 5'-déoxy-5'-alkylthio-nucléosides, en particulier les dérivés de l'adénosine et de l'inosine.

Les documents WO 98/05335 et WO 00/44750 décrivent des purines 2, 6, 9 tri-substituées, notamment utiles dans le traitement du cancer.

L'un des buts de la présente invention est de mettre à disposition des composés inhibiteurs de 5'-nucléotidases, pouvant notamment être utilisés dans le traitement du cancer.

Un autre but de l'invention est de mettre à disposition des composés inhibiteurs de 5'-nucléotidases qui, utilisés en association avec des analogues de nucléosides cytotoxiques et/ou de nucléobases habituellement utilisés en chimiothérapie anti-cancéreuse, potentialisent l'effet thérapeutique desdits analogues de nucléosides cytotoxiques et/ou de nucléobases.

La présente invention a ainsi pour objet un composé possédant un squelette de type 6-amino-purine caractérisé en ce qu'il présente la formule : 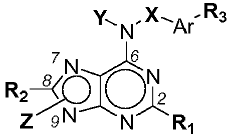 dans laquelle
R₁ et R₂, identiques ou différents, représentent indépendamment l'un de l'autre hydrogène, alkyle, alcényle, alcynyle, aryle, acyle (-COR₉), amino (-NH₂), alkylamino (-NHR₉), dialkylamino (-NR₉R'₉), acylamino (-NHCOR₉), diacylamino (-N(COR₉)(COR'₉)), trifluorométhyle (-CF₃), halogène, hydroxyle (-OH), alkoxy (-OR₉), thio (-SH), thioalkyle (-SR₉),
   avec R₉ et R'₉, identiques ou différents, représentant indépendamment l'un de l'autre alkyle, alcényle, alcynyle, aryle,
Z se situe sur l'une ou l'autre des positions N₇ ou N₉ de la purine, et représente hydrogène, alkyle, alcényle, alcynyle, aryle, -COR₉, halogène, -(CH₂)ₙ-OR₅, -(CH₂)ₙ₁-O-(CH₂)ₙ₂R₅, -(CH₂)_{n'}-COOR₅, -(CH₂)ₙ-P(=O)(OR₆)(OR₇), avec :
   n, n₁ et n₂, identiques ou différents, représentant indépendamment l'un de l'autre un entier allant de 1 à 10 et n' un entier allant de 0 à 10,
   R₅ représentant hydrogène, alkyle, alcényle, alcynyle, aryle, -COR₉,
   R₆ et R₇, identiques ou différents, représentant indépendamment l'un de l'autre hydrogène, alkyle, alcényle, alcynyle, aryle, -COR₉, un cation organique ou métallique,
X représente un radical divalent choisi parmi C=O, C=S, C=NR₈ ou SO₂, avec :
   R₈ représentant hydrogène, alkyle, alcényle, alcynyle, -OH, -OR₉,
Y a la même signification que R₅,
Ar représente un biphényle pouvant être substitué par un substituant R₃,
R₃ représente hydrogène, alkyle, alcényle, alcynyle, aryle, -NH₂, -NHR₉, -NR₉R'₉, -OH, -OR₉, aryloxy, benzyloxy (-OCH₂C₆H₅) ; un hétérocycle aromatique ou non à 5 ou 6 chaînons comprenant un ou plusieurs hétéroatomes choisis parmi N, O ou S, ledit hétérocycle à 5 ou 6 chaînons pouvant encore être substitué par un substituant R₄ avec :
   R₄ représentant hydrogène, alkyle, alcényle, alcynyle, aryle, -COR₉, -(CH₂)ₙ-OR₅, -(CH₂)ₙ₁-O-(CH₂)ₙ₂R₅, -(CH₂)_{n'}-COOR₅, -(CH₂)ₙ-P(=O)(OR₆)(OR₇),
   avec R₅, R₆, R₇, n, n₁, n₂ et n' tels que définis précédemment,
R₃ étant lié avec Ar en position ortho, méta ou para,
X étant lié avec Ar en position ortho, méta ou para,
à l'exception du composé 9*H*-purin-6-yl-[1,1'-biphényl]-4-carboxamide.

Dans la présente demande, le terme alkyle désigne un radical hydrocarboné, linéaire ou ramifié, ayant avantageusement de 1 à 12 atomes de carbone, et de préférence de 1 à 6 atomes de carbone tel que méthyle, éthyle, propyle, isopropyle, butyle, ter-butyle, pentyle, néopentyle ou n-hexyle.

Le terme alcényle désigne un radical hydrocarboné, linéaire ou ramifié, comportant une ou plusieurs doubles liaisons carbone-carbone, ayant avantageusement de 2 à 12 atomes de carbone, et de préférence de 2 à 6 atomes de carbone avec une ou deux doubles liaisons.

Le terme alcynyle désigne un radical hydrocarboné, linéaire ou ramifié, comportant une ou plusieurs triples liaisons carbone-carbone, ayant avantageusement de 2 à 12 atomes de carbone, et de préférence de 2 à 6 atomes de carbone avec une ou deux triples liaisons.

Le terme aryle désigne un système hydrocarboné aromatique mono-, bi- ou tricyclique, ayant de 6 à 18 atomes de carbone. A titre d'exemple on peut citer le phényle (C₆H₅), le benzyle (C₆H₅CH₂), le phénétyle (C₆H₅CH₂CH₂) le tolyl (C₆H₄CH₃), le xylyle (C₆H₃(CH₃)₂), le benzylidène (C₆H₅CH=CH), le benzoyle (C₆H₅CO), le biphényle (ou diphényle) (C₁₂H₉), le naphtyle (C₁₀H₇).

Ce terme aryle doit être différencié dans la présente demande de l'abréviation « Ar » représentée dans la formule générale ci-dessus ou dans la formule (I) définie ci-après, et qui désigne spécifiquement, d'une part un biphényle ou d'autre part un biphényle ou un naphtyle, chacun pouvant être substitué par un substituant R₃ avec R₃ tel que défini précédemment.

Les alkyles, alcényles, alcynyles, aryles tels que définis dans la demande peuvent en outre être substitués par un ou plusieurs substituants choisis parmi alkyle, alcényle, alcynyle, aryle, -NH₂, -NHR₉, -NR₉R'₉, -OH, -OR₉, aryloxy, benzyloxy (-OCH₂C₆H₅), un hétérocycle aromatique ou non à 5 ou 6 chaînons comprenant un ou plusieurs hétéroatomes choisis parmi N, O ou S.

Le terme halogène désigne un atome de fluor, de chlore, d'iode ou de brome.

A titre d'exemple de cation organique on pourra citer un cation ammonium, trialkylammonium ou pyridinium. A titre d'exemple de cation métallique on pourra citer un cation sodium (Na⁺), lithium (Li⁺) ou potassium (K⁺).

Dans le cas où R₆ et/ou R₇ désigne un cation organique ou métallique, le groupement -(CH₂)ₙ-P(=O)(OR₆)(OR₇) pourra être représenté par l'une des 3 formules suivantes :
-(CH₂)ₙ-P(=O)(O⁻ "Cation")(OR₇), -(CH₂)ₙ-P(=O)(OR₆)(O' "Cation") ou -(CH₂)ₙ-P(=O)(O' "Cation")(O⁻ "Cation"),
la désignation "Cation" impliquant forcément une charge positive.

Le terme aryloxy désigne un radical aryle lié à un atome d'oxygène.

Un hétérocycle à 5 chaînons, aromatique ou non, comprenant un ou plusieurs hétéroatomes choisis parmi N, O ou S désigne par exemple pyrrole, imidazole, furane, pyrroline, pyrrolidine, tétrahydrofurane, thiophène, tétrahydro-thiophène, pyrazole, oxazole, isoxazole, pyrazoline, imidazoline, pyrazolidine, imidazolidine, dioxolane, thiazole, thiazolidine, isoxazolidine, triazole, oxadiazole, furazane, thiadiazole, tétrazole.

Un hétérocycle à 6 chaînons, aromatique ou non, comprenant un ou plusieurs hétéroatomes choisis parmi N, O ou S désigne par exemple pyridine, pyrane, dihydro-pyrane, pipéridine, pyridazine, pyrimidine, pyrazine, oxazine, dioxine, pipérazine, morpholine, dioxane, thiazine, thiomorpholine, oxathiane, dithiane, triazine, trioxane, thiadiazine, dithiazine, trithiane.

Selon un mode de réalisation de l'invention, pour les composés tels que définis ci-dessus, le substituant R₃ représente un hydrogène ou un hétérocycle, aromatique ou non, à 5 ou 6 chaînons comprenant un ou plusieurs hétéroatomes choisis parmi N, O ou S, ledit hétérocyclique pouvant être substitué par un substituant R₄ tel que défini ci-dessus.

A titre d'exemple d'hétérocycle à 5 chaînons, on pourra citer un pyrrole ou un imidazole, ledit pyrrole ou imidazole pouvant être substitué par un substituant R₄ choisi parmi H ou -(CH₂)ₙ-P(=O)(OR₆)(OR₇) tel que défini ci-dessus.

Selon encore un mode de réalisation de l'invention, pour les composés tels que définis ci-dessus :
R₁ représente hydrogène, (-NH₂), (-NHR₉), (-NR₉R'₉), (-NHCOR₉), (-N(COR₉)(COR'₉)),
R₂ représente hydrogène, benzyle, phényle,
Z représente hydrogène, benzyle, phényle, -(CH₂)ₙ-OR₅, -(CH₂)ₙ₁-O-(CH₂)ₙ₂R₅, -(CH₂)_{n'}-COOR₅, -(CH₂)ₙ-P(=O)(OR₆)(OR₇) avec :
   n, n₁, n₂ et n', identiques ou différents, représentant indépendamment l'un de l'autre un entier égal à 1 ou 2,
   R₅ représentant hydrogène, éthyle, acétyle (-COCH₃), phényle,
   R₆ et R₇, identiques ou différents, représentant indépendamment l'un de l'autre hydrogène, méthyle, éthyle, un cation sodium (Na⁺), un cation lithium (Li⁺),
X représente C=O, SO₂,
Y représente hydrogène.

La présente invention a plus particulièrement pour objet un composé tel que défini ci-dessus, caractérisé en ce qu'il est choisi dans le groupe comprenant:
- 9*H*-purin-6-yl-[1,1'-biphényl]-2-carboxamide,
- 9*H*-purin-6-yl-[1,1'-biphényl]-3-carboxamide,
- 9*H*-purin-6-yl-[1,1'-biphényl]-3'-*N-*pyrrole-2-carboxamide,
- 9*H*-purin-6-yl-[1,1'-biphényl]-4'-*N*-pyrrole-2-carboxamide,
- 9*H*-purin-6-yl-[1,1'-biphényl]-3'-*N-*pyrrole-3-carboxamide,
- 9*H*-purin-6-yl-[1,1'-biphényl]-4'-*N-*pyrrole-3-carboxamide,
- 9*H*-purin-6-yl-[1,1'-biphényl]-3'-*N-*pyrrole-4-carboxamide,
- 9*H*-purin-6-yl-[1,1'-biphényl]-4'-*N-*pyrrole-4-carboxamide,
- 9*H*-purin-6-yl-[1,1'-biphényl]-3-'-*N-*imidazole-2-carboxamide,
- 9*H*-purin-6-yl-[1,1'-biphényl]-4'-*N*-imidazole-2-carboxamide,
- 9*H*-purin-6-yl-[1,1'-biphényl]-3'-*N-*imidazole-3-carboxamide,
- 9*H*-purin-6-yl-[1,1'-biphényl]-4'-*N*-imidazole-3-carboxamide,
- 9*H*-purin-6-yl-[1,1'-biphényl]-3',-*C₄*-imidazole-2-carboxamide,
- 9*H*-purin-6-yl-[1,1'-biphényl]-4'-*C₄*-imidazole-2-carboxamide,
- 9*H*-purin-6-yl-[1,1'-biphényl]-3'-*C₄*-imidazole-3-carboxamide,
- 9*H*-purin-6-yl-[1,1'-biphényl]-4'-*C₄*-imidazole-3-carboxamide,
- 9*H*-purin-6-yl-[1,1'-biphényl]-4'-*C₄*-(*N₁*-éthoxyphosphinylméthyl)imidazole-3-carboxamide,
- 7-(phénylméthyl)-*7H*-purin-6-yl-[1,1'-biphényl]-3-carboxamide,
- 9-(phénylméthyl)-9*H*-purin-6-yl-[1,1'-biphényl]-3-carboxamide,
- 7-[(phénylméthoxy)méthyl]-7*H*-purin-6-yl-[1,1'-biphényl]-3-carboxamide,
- 9-[(phénylméthoxy)méthyl]-9*H*-purin-6-yl-[1,1'-biphényl]-3-carboxamide,
- 9-[2-(acétyloxy)éthyl]-9*H*-purin-6-yl-[1,1'-biphényl]-3-carboxamide,
- 9-(2-hydroxyéthyl)-9*H*-purin-6-yl-[1,1'-biphényl]-3-carboxamide,
- 2-[(1,1'-biphényl)-3-carbonylamino-9*H*-purin-6-yl] acétate d'éthyle,
- acide 2-[(1,1'-biphényl)-3-carbonylamino-9*H-*purin-6-yl] acétique,
- [(1,1'-biphényl)-3-carbonylamino-9*H*-purin-6-yl] méthylphosphonate de diéthyle,
- acide [(1,1'-biphényl)-3-carbonylamino-9*H*-purin-6-yl] méthylphosphonique, et leurs mélanges.

L'invention concerne également un composé tel que défini ci-dessus pour son utilisation en tant que médicament, et de préférence pour son utilisation dans le traitement du cancer.

La présente invention a également pour objet un composé possédant un squelette de type 6-amino-purine caractérisé en ce qu'il présente la formule (I) : 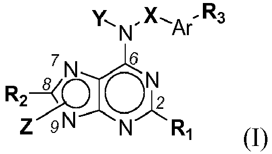 dans laquelle
R₁, R₂, R₃, X, Y et Z sont tels que définis précédemment,
Ar représente un biphényle ou un naphtyle, ledit biphényle ou naphtyle pouvant être substitué par un substituant R₃, avec R₃ étant tel que défini précédemment,
R₃ étant lié avec Ar en position ortho, méta ou para,
X étant lié avec Ar en position ortho, méta ou para,
ledit composé de formule (I) étant destiné à une utilisation dans le traitement du cancer.

Il doit être compris que, le composé 9*H*-purin-6-yl-[1,1'-biphényl]-4-carboxamide rentre bien dans la formule générale (I) telle que ci-dessus décrite et, selon l'invention, est destiné à une utilisation dans le traitement du cancer.

A titre d'exemples de composés de formule (I) pouvant avantageusement être utilisés dans le traitement du cancer, on pourra citer, outre les 27 composés ci-dessus spécifiquement cités:
- 9*H*-purin-6-yl-[1,1'-biphényl]-4-carboxamide,
- 9*H*-purin-6-yl-naphtalène-1-carboxamide,
- 9*H*-purin-6-yl-naphtalène-2-carboxamide,
- acide (naphtalène-1-carbonylamino-9*H*-purin-6-yl) méthylphosphonique,
- acide (naphtalène-2-carbonylamino-9*H*-purin-6-yl) méthylphosphonique,
- 9*H*-purin-6-yl-[1,1'-biphényl]-4-sulfonamide,
et leurs mélanges.

A titre d'exemples de cancers pouvant être traités à l'aide des composés de formule (I), on pourra citer ceux choisis parmi :
- les tumeurs solides, telles que les tumeurs des cellules glandulaires, cutanées, mésenchymateuses, génitales et neurologiques, ou
- les hémopathies aiguës (telles que les leucémies myéloblastiques aiguës et les leucémies lymphoblastiques aiguës), les syndromes myéloprolifératifs chroniques et les syndromes lymphoprolifératifs chroniques (tels que les lymphomes malins Hodgkiniens ou non Hodgkiniens), la leucémie lymphoïde chronique, la tricholeucocytose et le myélome multiple.

L'invention a aussi pour objet un composé de formule (I) pour une utilisation telle que définie ci-dessus, et plus particulièrement pour inhiber au moins une 5'-nucléotidase choisie parmi la 5'-nucléotidase cytosolique II (cN-II), la 5'-nucléotidase cytosolique IA (cN-IA), la 5'-nucléotidase cytosolique IB (cN-IB), la 5'-nucléotidase cytosolique IIIA (cN-IIIA), la 5'-nucléotidase cytosolique IIIB (cN-IIIB), l'ecto-5'-nucléotidase (eN, CD73), la 5'(3')-désoxynucléotidase cytosolique (cdN) ou la 5'(3')-désoxynucléotidase mitochondriale (mdN), et en particulier la 5'-nucléotidase cytosolique II.

Selon un mode de réalisation de l'invention, au moins un composé de formule (I) pourra être utilisé en association avec au moins un analogue de nucléoside et/ou au moins un analogue de nucléobase pour potentialiser l'effet anti-cancéreux dudit analogue de nucléoside et/ou dudit analogue de nucléobase. Ainsi, l'invention concerne un composé de formule (I) pour une utilisation telle que définie ci-dessus, caractérisé en ce que ledit composé de formule (I) est associé avec au moins un analogue de nucléoside et/ou au moins un analogue de nucléobase pour potentialiser l'effet anti-cancéreux dudit analogue de nucléoside et/ou dudit analogue de nucléobase.

L'analogue de nucléoside utilisé en association avec un composé de formule (I) pourra par exemple être choisi parmi la cladribine, la fludarabine, la clofarabine, la cytarabine, la gemcitabine, la nélarabine, la floxuridine ou la pentostatine.

L'analogue de nucléobase utilisé en association avec un composé de formule (I) pourra par exemple être choisi parmi le fluorouracile, la 6-mercaptopurine ou la 6-thioguanosine.

L'invention a encore pour objet une composition comprenant :
- au moins un composé de formule (I) tel que défini précédemment, en association avec :
- au moins un analogue de nucléoside choisi parmi la cladribine, la fludarabine, la clofarabine, la cytarabine, la gemcitabine, la nélarabine, la floxuridine ou la pentostatine, et/ou
- au moins un analogue de nucléobase choisi parmi le fluorouracile, la 6-mercaptopurine ou la 6-thioguanosine,
- et éventuellement au moins un excipient pharmaceutiquement acceptable.

Dans la suite, l'expression « composition de l'invention » désigne la composition telle que définie ci-dessus.

Les composés de formule (I) pouvant avantageusement être utilisés dans une composition de l'invention sont plus particulièrement les composés spécifiquement cités ci-dessus.

L'invention a encore pour objet la composition telle que ci-dessus définie pour son utilisation en tant que médicament, et de préférence dans le traitement du cancer.

Typiquement, la composition de l'invention telle que ci-dessus définie pourra être utilisée pour une administration simultanée, séparée ou séquentielle dans le traitement du cancer.

Les exemples de cancers pouvant plus particulièrement être traités à l'aide d'une composition de l'invention sont ceux décrits ci-dessus, à savoir :
- les tumeurs solides, telles que les tumeurs des cellules glandulaires, cutanées, mésenchymateuses, génitales et neurologiques, ou
- les hémopathies aiguës (telles que les leucémies myéloblastiques aiguës et les leucémies lymphoblastiques aiguës), les syndromes myéloprolifératifs chroniques et les syndromes lymphoprolifératifs chroniques (tels que les lymphomes malins Hodgkiniens ou non Hodgkiniens), la leucémie lymphoïde chronique, la tricholeucocytose et le myélome multiple.

La forme des compositions pharmaceutiques, leur voie d'administration, leur dosage et leur posologie dépendent naturellement de la sévérité de la pathologie, de son stade d'évolution, de l'âge, du sexe, du poids du sujet à traiter, etc.

L'homme du métier veillera donc à adapter les dosages en fonction du patient à traiter.

Les compositions pharmaceutiques selon l'invention peuvent être formulées pour une administration topique, orale, systémique, percutanée, transdermique, parentérale (à savoir intraveineuse, intramusculaire, sous-cutanée, intradermique) ou autre. Selon le mode d'administration, la composition selon l'invention peut se présenter sous toutes les formes galéniques.

Pour une administration par voie orale, la composition de l'invention peut se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée.

Pour une administration par voie parentérale, la composition selon l'invention peut se présenter notamment sous forme de solution aqueuse ou de dispersion de type lotion ou sérum, et sera conditionnée sous forme d'ampoules, de flacons de perfusion etc.

Ces compositions sont préparées selon les méthodes usuelles connues de l'homme de l'art.

L'invention concerne encore un composé de formule (I) tel que défini ci-dessus, en association avec au moins un composé choisi dans le groupe comprenant :
- un composé de formule (I),
- un analogue de nucléoside choisi parmi la cladribine, la fludarabine, la clofarabine, la cytarabine, la gemcitabine, la nélarabine, la floxuridine ou la pentostatine,
- un analogue de nucléobase choisi parmi le fluorouracile, la 6-mercaptopurine ou la 6-thioguanosine,
et leurs mélanges,
pour une administration simultanée, séparée ou séquentielle dans le traitement du cancer.

L'invention décrit encore l'utilisation d'un composé de formule (I) pour la fabrication d'un médicament, en particulier un médicament destiné au traitement du cancer.

L'invention décrit également des méthodes de traitement d'un sujet souffrant d'un cancer, notamment d'un cancer tel que défini précédemment, comprenant l'étape d'administration audit sujet d'une quantité thérapeutiquement efficace d'au moins un composé de formule (I) ou d'une composition de l'invention.

Par "quantité thérapeutiquement efficace" on entend une quantité suffisante pour traiter et/ou arrêter la progression dudit cancer.

L'invention décrit également un procédé de préparation d'un composé de formule (I), caractérisé en ce que :
- un composé 6-amino-purine de formule (II) : 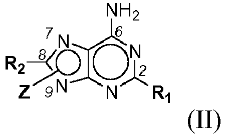 dans laquelle R₁, R₂ et Z sont tels que définis précédemment,
est mis à réagir avec :
- un composé de formule (III) :

   **R₁₀-Ar-R₃** (III)
dans laquelle Ar est un biphényle ou un naphtyle, ledit biphényle ou naphtyle étant substitué sur un de ses cycles par un substituant R₃ et sur son autre cycle par un substituant R₁₀,
à savoir le composé de formule (III) est représenté par l'une des formules ci-dessous : 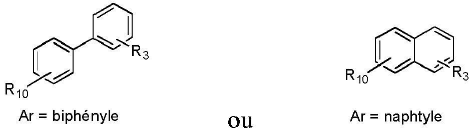 avec :
R₃ tel que défini précédemment,
R₁₀ représente -COOR₁₁, -COR₁₁, -SO₂R₁₁, avec R₁₁ représentant hydrogène, alkyle, aryle, ou halogène.

Plus particulièrement, lorsque Ar représente :
- un biphényle alors R₁₀ représente -COOR₁₁ ou -SO₂R₁₁,
- un naphtyle alors R₁₀ représente -COR₁₁,
avec R₁₁ tel que défini précédemment.

L'invention sera mieux comprise à la lumière des exemples purement illustratifs suivants.

La figure 1 illustre le schéma de synthèse général des composés de formule (I), dans laquelle R₁, R₂, X, Y, Z, Ar et R₃ sont tels que définis précédemment. Le composé de formule (III) est tel que défini précédemment.

Les figures 2 et 3 exemplifient la préparation d'un composé de formule (III) dans laquelle Ar représente un biphényle.

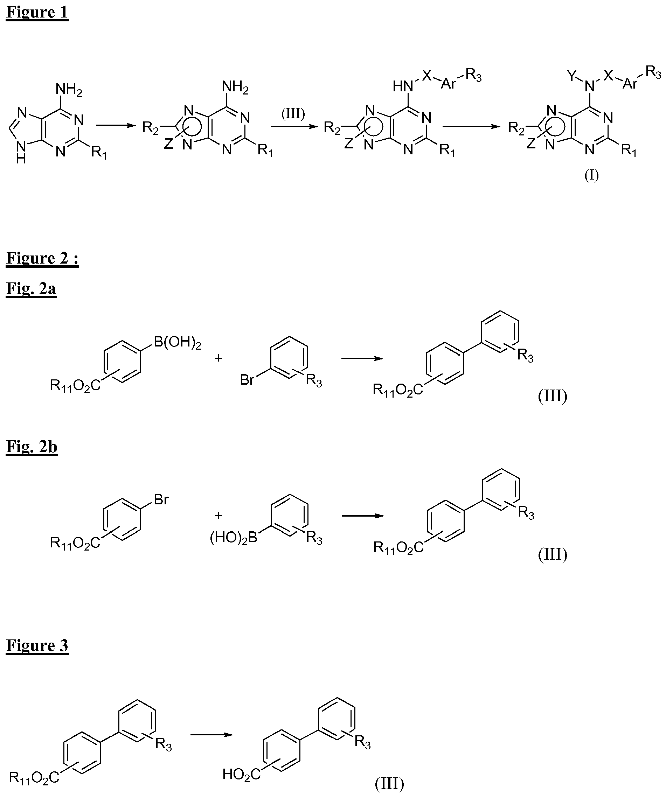

Plus particulièrement les figures 2a et 2b exemplifient deux voies de synthèse possibles pour préparer un composé de formule (III), dans laquelle le biphényle est substitué sur un de ses cycles par un substituant R₃ et sur son autre cycle par un substituant COOR₁₁. Dans la figure 3, le biphényle est substitué sur un de ses cycles par un substituant R₃ et sur son autre cycle par un substituant COOH.

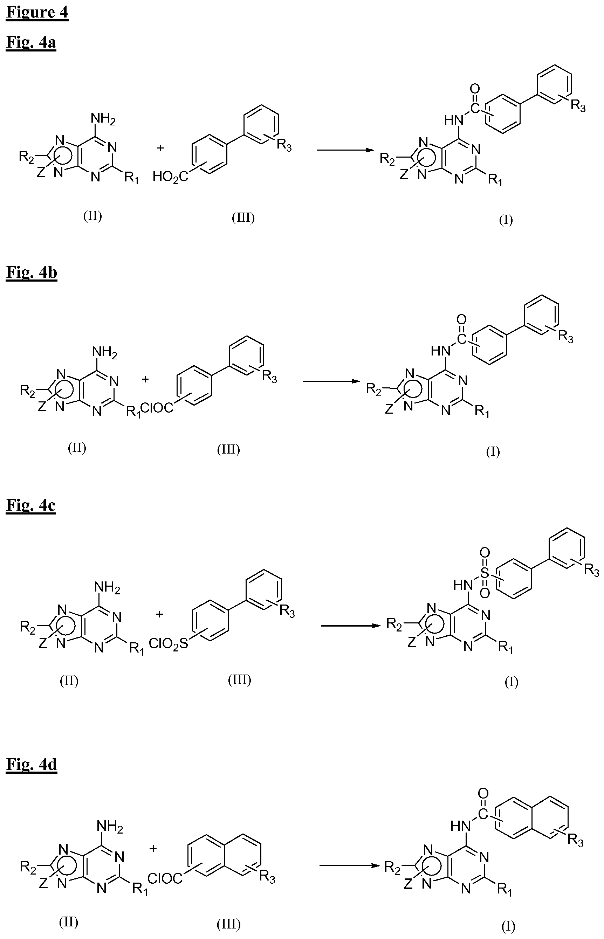

La figure 4 illustre un procédé de préparation de différents composés de formule (I) dans laquelle Y= H, X= (C=O) ou SO₂, Ar = biphényle ou naphtyle éventuellement substitué par un substituant R₃, avec R₁, R₂, Z et R₃ tels que définis précédemment.

Plus particulièrement, les figures 4a et 4b exemplifient la préparation d'un composé (I) de l'invention dans laquelle Y = H et X = (C=O) et Ar = biphényle.

La figure 4c exemplifie un procédé de préparation de composés (I) dans laquelle Y = H, X = SO₂ et Ar = biphényle.

La figure 4d exemplifie un procédé de préparation de composés (I) dans laquelle Y = H, X = (C=O) et Ar = naphtyle.

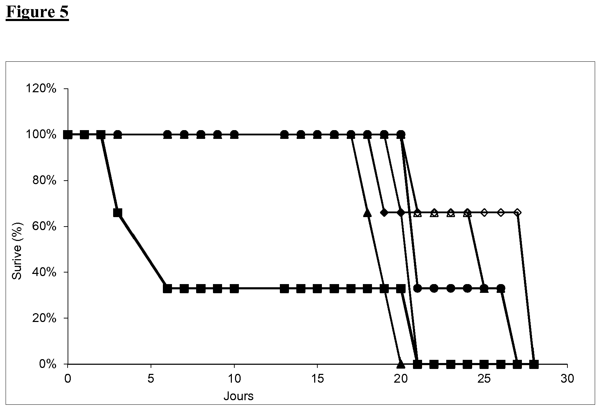

La figure 5 illustre la survie de souris B6D2F1 ayant reçu des cellules L1210 et traitées par :
- des cyclodextrines seules (■),
- de la fludarabine (100 mg/kg) (●),
- le composé (2) de l'invention seul (3,94 mg/kg) (▲) ou en association avec la fludarabine (100 mg/kg) (△),
- le composé (2) de l'invention seul (7,89 mg/kg) (◆) ou en association avec la fludarabine 100 mg/kg (◊).

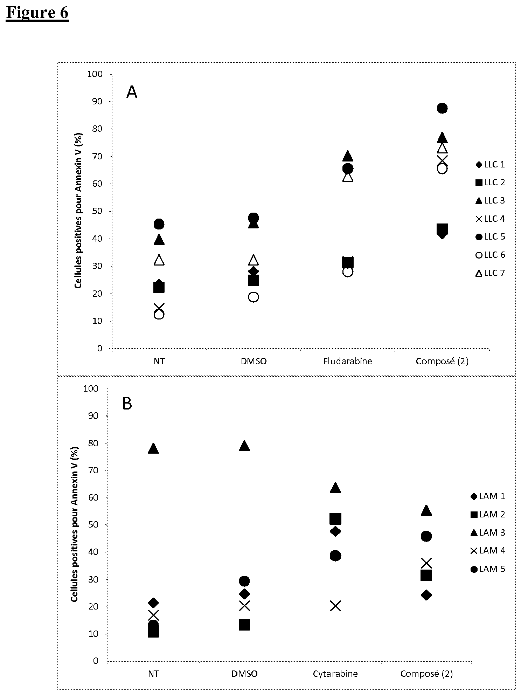

La figure 6 illustre le pourcentage de marquage Annexin V pour des cellules de patients LLC (Fig. 6A) et LAM (Fig. 6B) incubées en présence de DMSO, de fludarabine (10 µM), de cytarabine (100 µM) ou du composé (2) de l'invention (100 µM).

### Exemple 1 : illustration de 33 composés répondant à la formule (I) ci-dessus définie

Les tableaux 1 à 4 ci-dessous exemplifient 33 composés de formule (I) synthétisés par les Inventeurs, et désignés respectivement par (1) à (33) dans ce qui suit.

Dans les composés (1) à (18) du tableau 1 :
R₁ = R₂ = Y= Z= H, X= (C=O) (carbonyle), Ar = biphényle, ledit biphényle pouvant être substitué par un substituant R₃, ledit substituant R₃ étant choisi parmi hydrogène, pyrrole ou imidazole, ledit imidazole étant substitué ou non par un substituant R₄.

La dénomination chimique des composés (1) à (18) est respectivement la suivante :
- 9*H*-purin-6-yl-[1,1'-biphényl]-2-carboxamide (1),
- 9*H*-purin-6-yl-[1,1'-biphényl]-3-carboxamide (2),
- 9*H*-purin-6-yl-[1,1'-biphényl]-4-carboxamide (3),
- 9*H*-purin-6-yl-[1,1'-biphényl]-3'-*N*-pyrrole-2-carboxamide (4),
- 9*H*-purin-6-yl-[1,1'-biphényl]-4'-*N*-pyrrole-2-carboxamide (5),
- 9*H*-purin-6-yl-[1,1'-biphényl]-3'-*N*-pyrrole-3-carboxamide (6),
- 9*H*-purin-6-yl-[1,1'-biphényl]-4'-*N*-pyrrole-3-carboxamide (7),
- 9*H*-purin-6-yl-[1,1'-biphényl]-3'-*N*-pyrrole-4-carboxamide (8),
- 9*H*-purin-6-yl-[1,1'-biphényl]-4'-*N*-pyrrole-4-carboxamide (9),
- 9*H*-purin-6-yl-[1,1'-biphényl]-3'-*N-*imidazole-2-carboxamide (10),
- 9*H*-purin-6-yl-[1,1'-biphényl]-4'-*N*-imidazole-2-carboxamide (11),
- 9*H*-purin-6-yl-[1,1'-biphényl]-3'-*N-*imidazole-3-carboxamide (12),
- 9*H*-purin-6-yl-[1,1'-biphényl]-4'-*N*-imidazole-3-carboxamide (13),
- 9*H-*purin-6-yl-[1,1'-biphényl]-3'-*C₄*-imidazole-2-carboxamide (14),
- 9*H-*purin-6-yl-[1,1'-biphényl]-4'-*C₄*-imidazole-2-carboxamide (15),
- 9*H-*purin-6-yl-[1,1'-biphényl]-3'-*C₄*-imidazole-3-carboxamide (16),
- 9*H-*purin-6-yl-[1,1'-biphényl]-4'-*C₄*-imidazole-3-carboxamide (17),
- 9*H*-purin-6-yl-[1,1'-biphényl]-4'-*C₄*-(*N₁*-éthoxyphosphinylméthyl)imidazole-3-carboxamide (18).

L'orientation indiquée dans le tableau 1 (ortho, méta ou para) respectivement pour le groupement -NH-(C=O)- et pour le groupement R₃ concerne la position de chacun de ces groupements par rapport au biphényle.

**Tableau 1**

| -NH-(C=O)- orientation | R₃ / orientation | | R₄ | Composés (I) Numéros |
|---|---|---|---|---|
| *ortho* | -H | | / | (1) |
| *méta* | -H | | / | (2) |
| *para* | -H | | / | (3) |
| *ortho* | 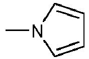 | *méta* | / | (4) |
| *ortho* | 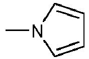 | *para* | / | (5) |
| *méta* | 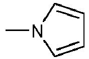 | *méta* | / | (6) |
| *méta* | 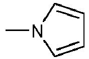 | *para* | / | (7) |
| *para* | 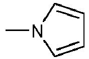 | *méta* | / | (8) |
| *para* | 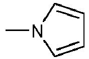 | *para* | / | (9) |
| *ortho* | 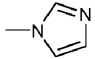 | *méta* | / | (10) |
| *ortho* | 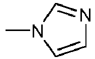 | *para* | / | (11) |
| *méta* | 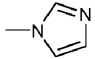 | *méta* | / | (12) |
| *méta* | 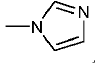 | *para* | / | (13) |
| *ortho* | 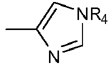 | *méta* | -H | (14) |
| *ortho* | 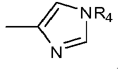 | *para* | -H | (15) |
| *méta* | 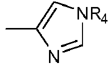 | *méta* | -H | (16) |
| *méta* | 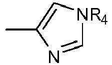 | *para* | -H | (17) |
| *méta* | 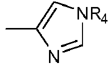 | *méta* | -CH₂-P(=O)(ONa)(OEt) | (18) |

Dans les composés (19) à (28) du tableau 2 :
R₁ = R₂ =Y= H, X= (C=O) (carbonyle), Ar = biphényle non substitué.

La dénomination chimique des composés (19) à (28) est respectivement la suivante :
- 7-(phénylméthyl)-7*H*-purin-6-yl-[1,1'-biphényl]-3-carboxamide (19),
- 9-(phénylméthyl)-9*H*-purin-6-yl-[1,1'-biphényl]-3-carboxamide (20),
- 7-[(phénylméthoxy)méthyl]-7*H*-purin-6-yl-[1,1'-biphényl]-3-carboxamide (21),
- 9-[(phénylméthoxy)méthyl]-9*H*-purin-6-yl-[1,1'-biphényl]-3-carboxamide (22),
- 9-[2-(acétyloxy)éthyl]-9*H*-purin-6-yl-[1,1'-biphényl]-3-carboxamide (23),
- 9-(2-hydroxyéthyl)-9*H*-purin-6-yl-[1,1'-biphényl]-3-carboxamide (24),
- 2-[(1,1'-biphényl)-3-carbonylamino-9*H*-purin-6-yl] acétate d'éthyle (25),
- acide 2-[(1,1'-biphényl)-3-carbonylamino-9*H*-purin-6-yl] acétique (26),
- [(1,1'-biphényl)-3-carbonylamino-9*H*-purin-6-yl] méthylphosphonate de diéthyle (27),
- acide [(1,1'-biphényl)-3-carbonylamino-9*H-*purin-6-yl] méthylphosphonique (28).

L'orientation du groupement -NH-(C=O)- indiquée (ortho, méta ou para) concerne la position dudit groupement par rapport au biphényle.

**Tableau 2**

| Z | -NH-(C=O)- orientation | Composés (I) Numéros |
|---|---|---|
| -CH₂Ph (N₇) | *méta* | (19) |
| -CH₂Ph (N₉) | *méta* | (20) |
| -CH₂-O-CH₂Ph (N₇) | *méta* | (21) |
| -CH₂-O-CH₂Ph (N₉) | *méta* | (22) |
| -(CH₂)₂-O-AC (N₉) | *méta* | (23) |
| -(CH₂)₂-OH (N₉) | *méta* | (24) |
| -CH₂-COOEt (N₉) | *méta* | (25) |
| -CH₂-COOH (N₉) | *méta* | (26) |
| -CH₂-P(=O)(OEt)₂ (N₉) | *méta* | (27) |
| -CH₂-P(=O)(OH)₂ (N₉) | *méta* | (28) |

N₇ ou N₉ indiquent la position du substituant Z sur l'azote respectivement en position 7 ou 9 du composé amino-purine (I).

Dans les composés (29) à (32) du tableau 3 :
R₁ = R₂ = Y = H, X= (C=O) (carbonyle), Ar = naphtyle non substitué.

La définition de Z est indiquée dans le tableau 3 pour chacun des composés (29) à (32).

La dénomination chimique des composés (29) à (32) est respectivement la suivante :
- 9*H*-purin-6-yl-naphtalène-1-carboxamide (29),
- 9*H*-purin-6-yl-naphtalène-2-carboxamide (30),
- acide (naphtalène-1-carbonylamino-9*H*-purin-6-yl) méthylphosphonique (31),
- acide (naphtalène-2-carbonylamino-9*H*-purin-6-yl) méthylphosphonique (32).

L'orientation du groupement -NH-(C=O)- indiquée (1 ou 2) dans le tableau 3 concerne la position dudit groupement par rapport au naphtyle.

**Tableau 3**

| Z | -NH-(C=O)- orientation | Composés (I) Numéros |
|---|---|---|
| H | *1* | (29) |
| H | *2* | (30) |
| -CH₂-P(=O)(OH)₂ (N₉) | *1* | (31) |
| -CH₂-P(=O)(OH)₂ (N₉) | *2* | (32) |

Dans le composé (33) du tableau 4 :
R₁ = R₂ =Y= H, X= (SO₂) (sulfonyle), Ar = biphényle non substitué.

La dénomination chimique du composé (33) est la suivante :
- 9*H*-purin-6-yl-[1,1'-biphényl]-4-sulfonamide (33).

**Tableau 4**

| Z | -NH-(SO₂)- orientation | Composé (I) Numéro |
|---|---|---|
| H | *para* | (33) |

L'orientation du groupement -NH-(SO₂)- indiquée (para) dans le tableau 4 concerne la position dudit groupement par rapport au biphényle.

### Exemple 2 : synthèse de composés de formule (I)

La figure 1 est un schéma de synthèse qui récapitule et généralise l'ensemble des étapes ci-dessous décrites.

### Synthèse des composés de formule (I) dans laquelle Y = H, X = (C=O) ou SO₂, Ar = biphényle ou naphtyle éventuellement substitué par un substituant R₃.

### 1) Préparation du composé biphényle de formule (III) dans lequel R₁₀ = COOR₁₁ (fig. 2a et 2b).

En fonction de la définition des substituants R₃ ou R₁₀, l'homme du métier adaptera les conditions expérimentales ci-dessous décrites.

### Synthèse du composé (III) dans lequel Ar = biphényle (fig. 2a)

Dans un ballon à fond rond à trois cols sont ajoutés, sous atmosphère d'argon, du Pd(PPh₃)₄ (palladium triphénylphosphine tétrakis) (0,1 éq.), du DMF (diméthylformamide) et du bromure de phényle substitué par un R₃ (1 éq.) (fig. 2a).

Sont ensuite successivement ajoutés du carbonate de potassium K₂CO₃ (3 éq.) et le dérivé benzèneboronique éthoxycarbonyle correspondant (1,7 éq.). Le mélange est agité sous argon à 100 °C, jusqu'à ce que la chromatographie sur couche mince (CCM) révèle que le produit de départ est consommé. Le mélange est refroidi à température ambiante, dilué avec de l'eau et le produit est extrait avec de l'acétate d'éthyle (EtOAc). Les phases organiques combinées sont lavées avec de la saumure, séchées sur du sulfate de sodium (Na₂SO₄) et filtrées. Le solvant est éliminé sous vide et le résidu est purifié par chromatographie sur colonne de gel de silice, élué par exemple avec un mélange dichlorométhane/méthanol (DCM/MeOH : 100/0 à 95/5) pour donner le composé ester méthylique ou ester éthylique attendu (III) sous forme d'une huile (fig. 2a).

### Synthèse du composé (III) dans lequel Ar = biphényle (fig. 2b)

Dans un ballon à fond rond à trois cols sont ajoutés, sous atmosphère d'argon, du Pd₂(dba)₃ (dipalladium tris(dibenzylideneacetone) (0,1 éq.), du DMF (diméthylformamide) et du bromure de benzoate de méthyle ou d'éthyle (1 éq.) (fig. 2b).

Sont ensuite successivement ajoutés du carbonate de potassium K₂CO₃ (3 éq.) et le dérivé benzèneboronique substitué par un R₃ (1,2 éq.). Le mélange est agité sous argon à 100 °C, jusqu'à ce que la chromatographie sur couche mince (CCM) révèle que le produit de départ est consommé. Le mélange est refroidi à température ambiante, dilué avec de l'eau, neutralisé par ajout d'une solution d'acide chlorhydrique (1M) et le produit est extrait avec de l'acétate d'éthyle (EtOAc). Les phases organiques combinées sont lavées avec de la saumure, séchées sur du sulfate de sodium (Na₂SO₄) et filtrées. Le solvant est éliminé sous vide et le résidu est purifié par chromatographie sur colonne de gel de silice, élué par exemple avec un mélange éther de pétrole/dichlorométhane (EP/DCM : 100/0 à 20/80) pour donner le composé ester méthylique ou ester éthylique attendu (III) sous forme d'une huile (fig. 2b).

### 2) Préparation du composé biphényle (III) dans lequel R₁₀ = COOH (fig. 3).

A une solution ou suspension du composé ester méthylique ou éthylique (III) tel qu'obtenu à l'étape précédente (point 1) ci-dessus) dans un mélange 1,4-dioxane/éthanol (2/1, v/v) est ajoutée goutte à goutte une solution d'hydroxyde de sodium (NaOH 2M) dans l'eau. Le mélange est agité à 50 °C jusqu'à ce que la CCM révèle l'achèvement de la réaction. Ensuite, une solution aqueuse d'acide chlorhydrique (HCl 1M) est ajoutée jusqu'à ce que le pH soit égal à 3 et le mélange est extrait avec EtOAc. Les phases organiques combinées sont lavées avec de la saumure, séchées sur Na₂SO₄ et filtrées. Le solvant est éliminé sous vide et le résidu est purifié par chromatographie sur colonne de gel de silice, élué par exemple avec DCM/MeOH (100/0 à 90/10) pour donner le composé acide carboxylique attendu (III) sous forme d'une poudre blanche (fig. 3).

### 3) Préparation d'un composé de formule (I) dans laquelle Y= H, X = CO et Ar = biphényle (fig. 4a et 4b).

En fonction de la définition des substituants R₁, R₂, Z et R₃, l'homme du métier choisira les produits de départ et adaptera si nécessaire les conditions expérimentales ci-dessous décrites.

### Synthèse du composé (I) telle qu'exemplifiée à la figure 4a

A une solution sous agitation du composé acide carboxylique (III) (1 éq.) tel qu'obtenu à l'étape précédente (point 2) ci-dessus) dans le DMF à température ambiante, est ajoutée un réactif de couplage approprié, comme par exemple un mélange diimidazole carbonyle/diméthylaminopyridine (CDI (2 éq.)/DMAP (0,2 éq.)). Après 5 minutes, le dérivé 2-amino-purine (2 éq.) de formule (II) est ajouté et le mélange est agité à 100°C jusqu'à la fin de la réaction. Ensuite, le solvant est éliminé sous vide et le résidu est purifié par chromatographie sur colonne de gel de silice, élué par exemple avec un mélange DCM/MeOH (100/0 à 90/10) pour donner le composé de formule (I) de l'invention avec Y= H et X = CO (fig. 4a).

### Synthèse du composé (I) telle qu'exemplifiée à la figure 4b

A une solution sous agitation d'un dérivé 2-amino-purine (1,25 éq.) de formule (II) dans la pyridine à température ambiante, est ajoutée le composé chlorure d'acide (III) commercial souhaité (1 éq.). Le mélange réactionnel est agité à 100 °C jusqu'à la fin de la réaction. Ensuite, le solvant est éliminé sous vide et le résidu est purifié par chromatographie sur colonne de gel de silice, élué par exemple avec DCM/MeOH (100/0 à 90/10) pour donner le composé (I°) attendu (fig. 4b).

### 4) Préparation d'un composé de formule (I) dans laquelle Y= H, X = SO₂ et Ar = biphényle (fig. 4c)

A une solution sous agitation d'un dérivé 2-amino-purine (1,25 éq.) de formule (II) dans la pyridine à température ambiante, est ajoutée le composé chlorure sulfuryle (III) commercial souhaité (1 éq.). Le mélange réactionnel est agité à 100 °C jusqu'à la fin de la réaction. Ensuite, le solvant est éliminé sous vide et le résidu est purifié par chromatographie sur colonne de gel de silice, élué par exemple avec DCM/MeOH (100/0 à 90/10) pour donner le composé (I°) attendu (fig. 4c).

### 5) Préparation d'un composé de formule (I) dans laquelle Y= H, X = CO et Ar = naphtyle (fig. 4d)

A une solution sous agitation d'un dérivé 2-amino-purine (1,25 éq.) de formule (II) dans la pyridine à température ambiante, est ajoutée le composé commercial chlorure d'acide (III) (avec Ar= naphtyle, voir Fig. 4d) souhaité (1 éq.). Le mélange réactionnel est agité à 100 °C jusqu'à la fin de la réaction. Ensuite, le solvant est éliminé sous vide et le résidu est purifié par chromatographie sur colonne de gel de silice, élué par exemple avec DCM/MeOH (100/0 à 90/10) pour donner le composé (I°) attendu (fig. 4d).

### Exemple 3 : détermination des propriétés inhibitrices et des activités cytotoxiques des composés (I) de l'invention

Les propriétés inhibitrices des composés (I) de l'invention ont été déterminées vis-à-vis de l'enzyme cible recombinante purifiée cN-II (détermination de l'inhibition de l'activité 5'-nucléotidasique).

Les activités cytotoxiques des composés de l'invention ont été évaluées dans différentes lignées cellulaires tumorales (détermination de l'IC₅₀).

Une sélection de ces données biologiques est présentée ci-après et concerne les composés de l'invention (I) pour lesquels une activité inhibitrice a été mesurée *in vitro* mais également en culture cellulaire : effet cytotoxique et/ou effet de synergie avec une des molécules anticancéreuses choisies parmi la cladribine, la fludarabine, la clofarabine, la cytarabine, la gemcitabine, la nélarabine, la floxuridine ou la pentostatine (à savoir analogues de nucléosides).

Les abréviations utilisées signifient :
**cN-II** : 5'-nucléotidase cytosolique II,
**RL** : Lignée cellulaire de lymphome folliculaire humain utilisée pour les tests de cytotoxicité (d'autres lignées telles que CCRF-CEM et MDA-MB-231 ont également été testées pour certains composés (I) de l'invention),
**MTT Synergie:** test d'évaluation de la synergie d'inhibition de la survie cellulaire entre les composés (I) de l'invention inhibiteurs de cN-II et l'agent anticancéreux connu (à savoir un analogue de nucléosides cytotoxique choisi parmi la cladribine, la fludarabine, la clofarabine, la cytarabine, la gemcitabine, la nélarabine, la floxuridine ou la pentostatine).

Le test MTT décrit ci-après permet d'évaluer si l'effet est de nature :
1) « additif » : effets indépendants des composés (I) de l'invention (inhibiteurs de la cN-II) et des anticancéreux connus,
2) « antagoniste » : effets opposés des composés (I) de l'invention et des anticancéreux connus ;
3) « synergique » : effet potentialisateur de l'anticancéreux connu par le composé (I) de l'invention, inhibiteur de cN-II.

C'est ce troisième point (effet synergique) qui est recherché dans ce qui suit.

### Procédure expérimentale du test d'inhibition de la cN-II :

L'activité de la cN-II est mesurée *in vitro* en suivant l'apparition du phosphate inorganique produit au cours de la réaction enzymatique. L'enzyme recombinante et purifiée cN-II est utilisée en présence de son substrat préférentiel, l'inosine 5'-monophosphate (IMP). Au cours de la réaction d'hydrolyse, de l'inosine et du phosphate inorganique sont produits à partir de l'IMP. Ce phosphate est ensuite dosé par une méthode colorimétrique utilisant le Vert de Malachite (kit vendu par la société Gentaur) : la lecture d'absorbance à 630 nm permet de quantifier le phosphate inorganique.

La même expérience est effectuée en présence des composés (I) de l'invention, inhibiteurs de 5'-nucléotidases (gamme de concentrations de 0 à 2 mM). Ce test de criblage « large » permet de déterminer le pourcentage d'inhibition de cN-II par les composés (I) dans la gamme de concentrations étudiées.

### Conditions expérimentales :

Les réactifs utilisés sont : tampon imidazole 50 mM, pH = 6.5, NaCl 500 mM et MgCl₂ 10 mM. La concentration en enzyme (cN-II) est de 0.1 µM et celle de l'IMP de 100 µM.

Une incubation à 37°C est réalisée pendant 2 à 5 minutes puis stoppée par l'addition du réactif Vert de Malachite qui contient un acide fort. Une gamme de concentration en phosphate est réalisée en parallèle pour la quantification du phosphate produit au cours de la réaction.

Pour les composés de l'invention qui ont démontré une inhibition forte avec ce premier test, un second test d'inhibition est réalisé par la mesure de la cinétique enzymatique. Ce test plus long permet de caractériser le mode d'inhibition et de déterminer la constante d'inhibition (Ki).

### Procédure expérimentale du test « MTT synergie » :

Pour les essais de synergie entre les composés (I) inhibiteurs de cN-II et les analogues de nucléosides cytotoxiques, les cellules sont ensemencées dans des plaques 96 puits contenant des concentrations variées de l'inhibiteur (I) seul, de l'analogue de nucléoside seul ou d'un mélange des deux composés à un ratio constant (proche du ratio des IC₅₀ pour chaque composé seul). Après 72 h d'incubation, les cellules vivantes sont quantifiées à l'aide du réactif MTT.

La concentration inhibitrice 50 (IC₅₀) et l'index de combinaison (CI₉₅) sont calculés avec le logiciel CompuSyn software 1.0 (ComboSyn, Inc., USA).

L'IC₅₀ correspond à la concentration d'un composé permettant une survie de 50% des cellules.

Le CI₉₅ est calculé selon la méthode de Chou et Talalay³ avec une formule prenant en compte les concentrations des deux composés et la fraction affectée à ces concentrations (*i.e*. les cellules mortes). Des valeurs de CI₉₅ inférieures à 0.9 indiquent une synergie entre les deux composés, des valeurs comprises entre 0.9 et 1.1 indiquent une additivité, et des valeurs supérieures à 1.1 indiquent un antagonisme selon les habitudes de la littérature⁴ et le manuel du logiciel (Users guide Compusyn). Cette méthode est la méthode de référence dans l'évaluation des interactions entre molécules ^{3; 5; 6; 7}.

Le tableau 5 ci-dessous regroupe l'ensemble des données expérimentales obtenues sur l'activité inhibitrice d'une vingtaine de composés de formule (I) de l'invention.

**Tableau 5**

| **Comp.(I) testés (N°)** | **Inhibition cN-II *in vitro (200 µM)*** | **Kᵢ (mM)** | **Force d'inhibition (Fort/Moyen/faible)** | **Lignée cancéreuse RL** | |
|---|---|---|---|---|---|
| | | | | **IC₅₀ (µM)** | **MTT synergie** |
| (1) | 17+/-5% | n.d.* | **f** / 70% inh. à 1 mM | 165 | Additif avec cladribine |
| (5) | 87 +/-3% | n.d. | **F** | 51 | Additif avec cladribine |
| (4) | 69 +/- 9 | n.a. | **F** | 25 | Additif avec cladribine |
| (11) | 0% | n.d. | **f/** 42% inh. à 0.8 mM | 51 | Antagoniste avec cladribine & clofarabine Additif avec fludarabine |
| (15) | 0% | n.d. | **f/** 58% inh. à 0.8 mM | 107 | n.d. |
| (10) | 13% | n.d. | **f** / 25% inh. à 0.8 mM | 168+/-56 | n.d. |
| (14) | 7-40% | n.d. | **f** / non reproductible | 215 +/-13 | n.d. |
| (6) | 56+/-13% | 1.53 | **F** | 11+/-4 | Antagoniste avec cladribine, clofarabine, fludarabine |
| (7) | 28% | n.d. | **M** | n.d | n.d. |
| (2) | 60+/-5% | 0.8 | **F**/ compétitif | 25 | Synergie avec cladribine & clofarabine |
| (13) | 10% | n.d. | **f** | 203 | n.d. |
| (12) | 39+/-11% | n.d. | **f** | 34 | Synergie avec cladribine & clofarabine Antagoniste avec fludarabine |
| (16) | 47+/-12% | n.d. | **f** | 53 | Synergie avec clofarabine Antagoniste avec fludarabine |
| (17) | 21 +/- 10% | n.d. | **f** | 5 | Synergie avec clofarabine Antagoniste avec fludarabine |
| (19) | 68 +/-15% | n.d. | **F** | 60 | Synergie avec cladribine & fludarabine |
| (20) | 58 +/-19 % | n.d. | **F** | 51 | Antagoniste avec cladribine & clofarabine Synergie avec fludarabine |
| (22) | 43+/-7% | n.d. | **M** | 188 +/-33 | n.d. |
| (21) | 50 +/-2% | n.d. | **M** | 36 +/- 5 | Synergie avec cladribine & clofarabine Additif avec fludarabine |
| (3) | 60 +/- 5% | 0.8 | **F** / compétitif | 128 | n.d. |
| (9) | 0% | n.d. | Pas d'effet / Insoluble | 98 | Synergie avec cladribine Antagoniste avec fludarabine |
| (29) | 40+/-10% | n.d. | **M** | 130 | n.d. |
| (30) | 35+/-10% | n.d. | **M** | 58 | n.d. |
| (31) | 10% | n.d. | **f** | 250 | n.d. |
| (32) | 10% | n.d. | **f** | 145 | n.d. |
| (33) | 70+/-5% | n.d. | **F** | 195 | n.d. |

| | | | | | |
|---|---|---|---|---|---|
| *n.d. : non déterminé ; n.a. : non applicable car composé insoluble dans le tampon de réaction. | | | | | |

### Conclusion

Les composés (2), (9), (12), (16), (17), (19), (20) et (21) montrent un effet synergique avec au moins un des trois agents anti-cancéreux de l'art antérieur.

Les composés (2), (4), (6), (12), (17) et (21) montrent également une activité cytotoxique intrinsèque sur le modèle cellulaire utilisé avec des IC₅₀ de l'ordre de quelques micromolaires à quelques dizaines de micromolaires.

### Exemple 4 : évaluation de l'activité antitumorale in vivo et cytotoxique ex vivo d'un composé de l'invention

Les propriétés antitumorales du composé (2) de l'invention, à savoir le 9*H*-purin-6-yl-[1,1'-biphényl]-3-carboxamide, ont été déterminées dans un modèle syngénique de tumeur intrapéritonéale chez la souris.

### Procédure expérimentale de l'évaluation in vivo :

Afin d'obtenir des solutions du composé (2) à des concentrations compatibles avec les évaluations in vivo, le composé (2) est solubilisé à 10 mM à l'aide de 2,6-diO-méthyle-béta-cyclodextrines. Des cellules de leucémie murine L1210 (1 million) sont injectées dans la cavité intrapéritonéale de souris B6D2F1 (trois souris par groupe) âgées de quatre semaines au jour 1, et les souris sont traitées aux jours 2, 4, 7, 9 et 11 par la fludarabine (100 mg/kg), le composé (2) (7,89 mg/kg ou 3,94 mg/kg), une association de fludarabine et du composé (2) ou une solution de cyclodextrines seules. La survie des souris est utilisée comme point final de l'expérience (voir figure 5).

### Conclusion :

Une augmentation de la dose du composé (2) de 3,94 à 7,89 mg/kg permet de prolonger la survie des souris indiquant un effet dose dans cette gamme.

L'association entre le composé (2), en particulier à 7,89 mg/kg, et la fludarabine à 100 mg/kg permet de prolonger la survie des souris par rapport à la fludarabine seule, indiquant un effet potentialisateur de cette association.

### Procédure expérimentale de l'évaluation ex vivo :

Du sang périphérique a été récupéré de patients atteints de leucémie lymphoblastique chronique (LLC) ou de leucémie aiguë myéloïde (LAM) sur des tubes héparinés. Après lyse des globules rouges, le sang est incubé pendant 24 heures en présence de DMSO, de fludarabine 10 µM, de cytarabine 100 µM ou du composé de l'invention (2) 100 µM avant détermination de l'induction de l'apoptose et de la mort cellulaire avec les marqueurs Annexin V et iodure de propidium par cytométrie en flux. Les cellules ayant subi un effet des incubations sont marquées en Annexin V seul ou avec l'iodure de propidium (voir figure 6).

### Conclusion :

Tous les échantillons LLC sont plus sensibles à une incubation de 100 µM du composé (2) de l'invention qu'à une incubation de 10 µM de fludarabine (65,3% versus 45,8%), ce qui indique une bonne cytotoxicité du composé (2) pour ces cellules.

Pour les échantillons LAM, deux sur cinq sont plus sensibles à 100 µM du composé (2) qu'à 100 µM de cytarabine, avec une moyenne sur les cinq échantillons en faveur de la cytarabine (44,5% vs. 38,6%).

### CONCLUSION GENERALE

L'originalité de l'invention repose sur la nature de la cible pharmacologique (cytosolique et non membranaire). Aucun inhibiteur de 5'-nucléotidases intracellulaire (cytosolique) n'est à ce jour décrit dans le traitement de pathologies humaines.

L'association inédite des composés de l'invention, inhibiteurs de 5'-nucléotidases, et en particulier de la cN-II, à des analogues de nucléosides cytotoxiques connus à ce jour, permet d'accroître l'efficacité de cette classe médicamenteuse par plusieurs mécanismes : (1) par l'inhibition intrinsèque de la cN-II induisant un mécanisme d'apoptose ; (2) en augmentant la concentration intracellulaire des formes phosphorylées (nucléotidiques) de l'analogue nucléosidique, formes responsables de son activité antiproliférative ; (3) en permettant de répondre à certains mécanismes de résistance cellulaire associés à la surexpression de la cN-II.

### REFERENCES BIBLIOGRAPHIQUES

1. Fakhoury M., De Beaumais T., Médard Y. & Jacqz-Aigrain E. (2010). Suivi thérapeutique pharmacologique des 6-thioguanine nucléotides dans les leucémies aiguës lymphoblastiques de l'enfant: intérêt et limites. Thérapie, 65 (3): 187-193.
2. a) Tzoneva, G.; Perez-Garcia, A.; Carpenter, Z.; Khiabanian, H.; Tosello, V.; Allegretta, M.; Paietta, E.; Racevskis, J.; Rowe, J.M.; Tallman, M.S.; Paganin, M.; Basso, G.; Hof, J.; Kirschner-Schwabe, R.; Palomero, T.; Rabadan, R. & Ferrando, A. (2013). Activating mutations in the NT5C2 nucleotidase gene drive chemotherapy résistance in relapsed ALL. Nat Med, 19 (3), 368-71. b) Meyer, J.A.; Wang, J.; Hogan, L.E.; Yang, J.J.; Dandekar, S.; Patel, J.P.; Tang, Z.; Zumbo, P.; Li, S.; Zavadil, J.; Levine, R.L.; Cardozo, T.; Hunger, S.P.; Raetz, E.A.; Evans, W.E.; Morrison, D.J.; Mason, C.E. & Carroll, W.L. (2013). Relapse-specific mutations in NT5C2 in childhood acute lymphoblastic leukemia. Nat Genet, 45 (3), 290-4.
3. Chou, T. C. & Talalay, P. (1984). Quantitative analysis of dose-effect relationships: the combined effects of multiple drugs or enzyme inhibitors. Adv Enzyme Regul 22, 27-55.
4. Bijnsdorp, I. V., Giovannetti, E. & Peters, G. J. Analysis of drug interactions. Methods Mol Biol 731, 421-34.
5. Chou, T. C. (2006). Theoretical basis, experimental design, and computerized simulation of synergism and antagonism in drug combination studies. Pharmacol Rev 58, 621-81.
6. Chou, T. C. (2010). Drug combination studies and their synergy quantification using the Chou-Talalay method. Cancer Res 70, 440-6.
7. Tallarida, R. J. (2006). An overview of drug combination analysis with isobolograms. J Pharmacol Exp Ther 319, 1-7.

## Revendications

1. Composé possédant un squelette de type 6-amino-purine **caractérisé en ce qu'**il présente la formule : 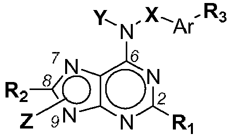 dans laquelle
R₁ et R₂, identiques ou différents, représentent indépendamment l'un de l'autre hydrogène, alkyle, alcényle, alcynyle, aryle, acyle (-COR₉), amino (-NH₂), alkylamino (-NHR₉), dialkylamino (-NR₉R'₉), acylamino (-NHCOR₉), diacylamino (-N(COR₉)(COR'₉)), trifluorométhyle (-CF₃), halogène, hydroxyle (-OH), alkoxy (-OR₉), thio (-SH), thioalkyle (-SR₉),
avec R₉ et R'₉, identiques ou différents, représentant indépendamment l'un de l'autre alkyle, alcényle, alcynyle, aryle,
Z se situe sur l'une ou l'autre des positions N₇ ou N₉ de la purine, et représente hydrogène, alkyle, alcényle, alcynyle, aryle, -COR₉, halogène, -(CH₂)ₙ-OR₅, -(CH₂)ₙ₁-O-(CH₂)ₙ₂R₅, -(CH₂)ₙ,-COOR₅, -(CH₂)ₙ-P(=O)(OR₆)(OR₇), avec :
n, n₁ et n₂, identiques ou différents, représentant indépendamment l'un de l'autre un entier allant de 1 à 10 et n' un entier allant de 0 à 10,
R₅ représentant hydrogène, alkyle, alcényle, alcynyle, aryle, -COR₉,
R₆ et R₇, identiques ou différents, représentant indépendamment l'un de l'autre hydrogène, alkyle, alcényle, alcynyle, aryle, -COR₉, un cation organique ou métallique,
X représente un radical divalent choisi parmi C=O, C=S, C=NR₈ ou SO₂, avec :
R₈ représentant hydrogène, alkyle, alcényle, alcynyle, -OH, -OR₉,
Y a la même signification que R₅,
Ar représente un biphényle pouvant être substitué par un substituant R₃,
R₃ représente hydrogène, alkyle, alcényle, alcynyle, aryle, -NH₂, -NHR₉, -NR₉R'₉, -OH, -OR₉, aryloxy, benzyloxy (-OCH₂C₆H₅) ; un hétérocycle aromatique ou non à 5 ou 6 chaînons comprenant un ou plusieurs hétéroatomes choisis parmi N, O ou S, ledit hétérocycle à 5 ou 6 chaînons pouvant encore être substitué par un substituant R₄ avec :
R₄ représentant hydrogène, alkyle, alcényle, alcynyle, aryle, -COR₉, -(CH₂)ₙ-OR₅, -(CH₂)ₙ₁-O-(CH₂)ₙ₂R₅, -(CH₂)_{n'}-COOR₅, -(CH₂).-P(=O)(OR₆R₇),
R₃ étant lié avec Ar en position ortho, méta ou para,
X étant lié avec Ar en position ortho, méta ou para,
à l'exception du composé 9*H*-purin-6-yl-[1,1'-biphényl]-4-carboxamide.

2. Composé selon la revendication 1, **caractérisé en ce que** le substituant R₃ représente un hydrogène ou un hétérocycle, aromatique ou non, à 5 ou 6 chaînons comprenant un ou plusieurs hétéroatomes choisis parmi N, O ou S, ledit hétérocyclique pouvant être substitué par un substituant R₄ tel que défini à la revendication 1.

3. Composé selon la revendication 1 ou 2, **caractérisé en ce que** l'hétérocycle à 5 chaînons représente un pyrrole ou un imidazole, ledit pyrrole ou imidazole pouvant être substitué par un substituant R₄ choisi parmi hydrogène ou -(CH₂)ₙ-P(=O)(OR₆)(OR₇) tel que défini à la revendication 1.

4. Composé selon l'une quelconque des revendications 1 à 3, **caractérisé en ce que** :
R₁ représente hydrogène, (-NH₂), (-NHR₉), (-NR₉R'₉), (-NHCOR₉), (-N(COR₉)(COR'₉)),
R₂ représente hydrogène, benzyle, phényle,
Z représente hydrogène, benzyle, phényle, -(CH₂)ₙ-OR₅, -(CH₂)ₙ₁-O-(CH₂)ₙ₂R₅, -(CH₂)_{n'}-COOR₅, -(CH₂)ₙ-P(=O)(OR₆)(OR₇) avec :
n, n₁, n₂ et n', identiques ou différents, représentant indépendamment l'un de l'autre un entier égal à 1 ou 2,
R₅ représentant hydrogène, éthyle, acétyle (-COCH₃), phényle,
R₆ et R₇, identiques ou différents, représentant indépendamment l'un de l'autre
hydrogène, méthyle, éthyle, un cation sodium (Na⁺), un cation lithium (Li⁺),
X représente C=O, SO₂,
Y représente hydrogène.

5. Composé selon l'une quelconque des revendications 1 à 4, **caractérisé en ce qu'**il est choisi dans le groupe comprenant:
- 9*H*-purin-6-yl-[1,1'-biphényl]-2-carboxamide,
- 9*H*-purin-6-yl-[1,1'-biphényl]-3-carboxamide,
- 9*H*-purin-6-yl-[1,1'-biphényl]-3'-*N*-pyrrole-2-carboxamide,
- 9*H*-purin-6-yl-[1,1'-biphényl]-4'-*N*-pyrrole-2-carboxamide,
- 9*H*-purin-6-yl-[1,1'-biphényl]-3'-*N*-pyrrole-3-carboxamide,
- 9*H*-purin-6-yl-[1,1'-biphényl]-4'-*N*-pyrrole-3-carboxamide,
- 9*H*-purin-6-yl-[1,1'-biphényl]-3'-*N*-pyrrole-4-carboxamide,
- 9*H*-purin-6-yl-[1,1'-biphényl]-4'-*N*-pyrrole-4-carboxamide,
- 9*H*-purin-6-yl-[1,1'-biphényl]-3'-*N*-imidazole-2-carboxamide,
- 9*H*-purin-6-yl-[1,1'-biphényl]-4'-*N*-imidazole-2-carboxamide,
- 9*H*-purin-6-yl-[1,1'-biphényl]-3 '-*N-*imidazole-3-carboxamide,
- 9*H*-purin-6-yl-[1,1'-biphényl]-4'-*N*-imidazole-3-carboxamide,
- 9*H*-purin-6-yl-[1,1'-biphényl]-3'-*C₄*-imidazole-2-carboxamide,
- 9*H*-purin-6-yl-[1,1'-biphényl]-4'-*C₄*-imidazole-2-carboxamide,
- 9*H*-purin-6-yl-[1,1'-biphényl]-3'-*C₄*-imidazole-3-carboxamide,
- 9*H*-purin-6-yl-[1,1'-biphényl]-4'-*C₄*-imidazole-3-carboxamide,
- 9*H*-purin-6-yl-[1,1'-biphényl]-4'-*C₄*-(*N₁*-éthoxyphosphinylméthyl)imidazole-3-carboxamide,
- 7-(phénylméthyl)-7*H*-purin-6-yl-[1,1'-biphényl]-3-carboxamide,
- 9-(phénylméthyl)-9*H*-purin-6-yl-[1,1'-biphényl]-3-carboxamide,
- 7-[(phénylméthoxy)méthyl]-7*H*-purin-6-yl-[1,1'-biphényl]-3-carboxamide,
- 9-[(phénylméthoxy)méthyl]-9*H*-purin-6-yl-[1,1'-biphényl]-3-carboxamide,
- 9-[2-(acétyloxy)éthyl]-9*H*-purin-6-yl-[1,1'-biphényl]-3-carboxamide,
- 9-(2-hydroxyéthyl)-9*H*-purin-6-yl-[1,1'-biphényl]-3-carboxamide,
- 2-[(1,1'-biphényl)-3-carbonylamino-9*H*-purin-6-yl] acétate d'éthyle,
- acide 2-[(1,1'-biphényl)-3-carbonylamino-9*H*-purin-6-yl] acétique,
- [(1,1'-biphényl)-3-carbonylamino-9*H*-purin-6-yl] méthylphosphonate de diéthyle,
- acide [(1,1'-biphényl)-3-carbonylamino-9*H*-purin-6-yl] méthylphosphonique, et leurs mélanges.

6. Composé possédant un squelette de type 6-amino-purine **caractérisé en ce qu'**il présente la formule (I) : 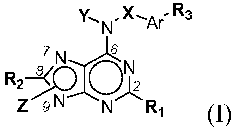 dans laquelle
R₁, R₂, R₃, X, Y et Z sont tels que définis à l'une quelconque des revendications 1 à 5,
Ar représente un biphényle ou un naphtyle, ledit biphényle ou naphtyle pouvant être substitué par un substituant R₃,
R₃ étant lié avec Ar en position ortho, méta ou para,
X étant lié avec Ar en position ortho, méta ou para,
ledit composé étant destiné à une utilisation dans le traitement du cancer.

7. Composé de formule (I) pour une utilisation selon la revendication 6, **caractérisé en ce qu'**il est choisi dans le groupe comprenant:
- 9*H*-purin-6-yl-[1,1'-biphényl]-2-carboxamide,
- 9*H*-purin-6-yl-[1,1'-biphényl]-3-carboxamide,
- 9*H*-purin-6-yl-[1,1'-biphényl]-4-carboxamide,
- 9*H*-purin-6-yl-[1,1'-biphényl]-3'-*N*-pyrrole-2-carboxamide,
- 9*H*-purin-6-yl-[1,1'-biphényl]-4'-*N*-pyrrole-2-carboxamide,
- 9*H*-purin-6-yl-[1,1'-biphényl]-3'-*N*-pyrrole-3-carboxamide,
- 9*H*-purin-6-yl-[1,1'-biphényl]-4'-*N*-pyrrole-3-carboxamide,
- 9*H*-purin-6-yl-[1,1'-biphényl]-3'-*N*-pyrrole-4-carboxamide,
- 9*H*-purin-6-yl-[1,1'-biphényl]-4'-*N*-pyrrole-4-carboxamide,
- 9*H*-purin-6-yl-[1,1'-biphényl]-3'-*N*-imidazole-2-carboxamide,
- 9*H*-purin-6-yl-[1,1'-biphényl]-4'-*N*-imidazole-2-carboxamide,
- 9*H*-purin-6-yl-[1,1'-biphényl]-3'-*N-*imidazole-3-carboxamide,
- 9*H*-purin-6-yl-[1,1'-biphényl]-4'-*N*-imidazole-3-carboxamide,
- 9*H*-purin-6-yl-[1,1'-biphényl]-3'-*C₄*-imidazole-2-carboxamide,
- 9*H*-purin-6-yl-[1,1'-biphényl]-4'-*C₄*-imidazole-2-carboxamide,
- 9*H*-purin-6-yl-[1,1'-biphényl]-3'-*C₄*-imidazole-3-carboxamide,
- 9*H*-purin-6-yl-[1,1'-biphényl]-4'-*C₄*-imidazole-3-carboxamide,
- 9*H*-purin-6-yl-[1,1'-biphényl]-4'-*C₄*-(*N₁*-éthoxyphosphinylméthyl)imidazole-3-carboxamide,
- 7-(phénylméthyl)-7*H*-purin-6-yl-[1,1'-biphényl]-3-carboxamide,
- 9-(phénylméthyl)-9*H*-purin-6-yl-[1,1'-biphényl]-3-carboxamide,
- 7-[(phénylméthoxy)méthyl]-7*H*-purin-6-yl-[1,1'-biphényl]-3-carboxamide,
- 9-[(phénylméthoxy)méthyl]-9*H*-purin-6-yl-[1,1'-biphényl]-3-carboxamide,
- 9-[2-(acétyloxy)éthyl]-9*H*-purin-6-yl-[1,1'-biphényl]-3-carboxamide,
- 9-(2-hydroxyéthyl)-9*H*-purin-6-yl-[1,1'-biphényl]-3-carboxamide,
- 2-[(1,1'-biphényl)-3-carbonylamino-9*H*-purin-6-yl] acétate d'éthyle,
- acide 2-[(1,1'-biphényl)-3-carbonylamino-9*H*-purin-6-yl] acétique,
- [(1,1'-biphényl)-3-carbonylamino-9*H*-purin-6-yl] méthylphosphonate de diéthyle,
- acide [(1,1'-biphényl)-3-carbonylamino-9*H*-purin-6-yl] méthylphosphonique,
- 9*H*-purin-6-yl-naphtalène-1-carboxamide,
- 9*H*-purin-6-yl-naphtalène-2-carboxamide,
- acide (naphtalène-1-carbonylammo-9*H*-purin-6-yl) méthylphosphonique,
- acide (naphtalène-2-carbonylamino-9*H*-purin-6-yl) méthylphosphonique,
- 9*H*-purin-6-yl-[1,1'-biphényl]-4-sulfonamide,
et leurs mélanges.

8. Composé de formule (I) pour une utilisation selon la revendication 6 ou 7, pour inhiber au moins une 5'-nucléotidase choisie parmi la 5'-nucléotidase cytosolique II (cN-II), la 5'-nucléotidase cytosolique IA (cN-IA), la 5'-nucléotidase cytosolique IB (cN-IB), la 5'-nucléotidase cytosolique IIIA (cN-IIIA), la 5'-nucléotidase cytosolique IIIB (cN-IIIB), l'ecto-5'-nucléotidase (eN, CD73), la 5'(3')-désoxynucléotidase cytosolique (cdN) ou la 5'(3')-désoxynucléotidase mitochondriale (mdN).

9. Composé de formule (I) pour une utilisation selon l'une quelconque des revendications 6 à 8, **caractérisé en ce qu'**il est associé avec au moins un analogue de nucléoside et/ou au moins un analogue de nucléobase pour potentialiser l'effet anti-cancéreux dudit analogue de nucléoside et/ou de nucléobase.

10. Composé de formule (I) pour une utilisation selon la revendication 9, dans laquelle :
- l'analogue de nucléoside est choisi parmi la cladribine, la fludarabine, la clofarabine, la cytarabine, la gemcitabine, la nélarabine, la floxuridine ou la pentostatine,
- l'analogue de nucléobase est choisi parmi le fluorouracile, la 6-mercaptopurine ou la 6-thioguanosine.

11. Composition comprenant :
- au moins un composé de formule (I) : 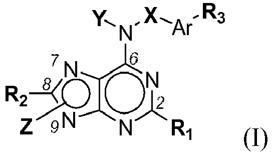 dans laquelle
R₁, R₂, R₃, X, Y et Z sont tels que définis à l'une quelconque des revendications 1 à 5,
Ar représente un biphényle ou un naphtyle, ledit biphényle ou naphtyle pouvant être substitué par un substituant R₃,
R₃ étant lié avec Ar en position ortho, méta ou para,
X étant lié avec Ar en position ortho, méta ou para,
en association avec :
- au moins un analogue de nucléoside choisi parmi la cladribine, la fludarabine, la clofarabine, la cytarabine, la gemcitabine, la nélarabine, la floxuridine ou la pentostatine, et/ou
- au moins un analogue de nucléobase choisi parmi le fluorouracile, la 6-mercaptopurine ou la 6-thioguanosine,
- et éventuellement au moins un excipient pharmaceutiquement acceptable.

12. Composition selon la revendication 11, dans laquelle le composé de formule (I) est choisi dans le groupe comprenant :
- 9*H*-purin-6-yl-[1,1'-biphényl]-2-carboxamide,
- 9*H*-purin-6-yl-[1,1'-biphényl]-3-carboxamide,
- 9*H*-purin-6-yl-[1,1'-biphényl]-4-carboxamide,
- 9*H*-purin-6-yl-[1,1'-biphényl]-3'-*N*-pyrrole-2-carboxamide,
- 9*H*-purin-6-yl-[1,1'-biphényl]-4'-*N*-pyrrole-2-carboxamide,
- 9*H*-purin-6-yl-[1,1'-biphényl]-3'-*N*-pyrrole-3-carboxamide,
- 9*H*-purin-6-yl-[1,1'-biphényl]-4'-*N*-pyrrole-3-carboxamide,
- 9*H*-purin-6-yl-[1,1'-biphényl]-3'-*N*-pyrrole-4-carboxamide,
- 9*H*-purin-6-yl-[1,1'-biphényl]-4'-*N*-pyrrole-4-carboxamide,
- 9*H*-purin-6-yl-[1,1 '-biphényl]-3'-*N*-imidazole-2-carboxamide,
- 9*H*-purin-6-yl-[1,1'-biphényl]-4'-*N*-imidazole-2-carboxamide,
- 9*H*-purin-6-yl-[1,1'-biphényl]-3'-*N-*imidazole-3-carboxamide,
- 9*H*-purin-6-yl-[1,1'-biphényl]-4'-*N*-imidazole-3-carboxamide,
- 9*H*-purin-6-yl-[1,1'-biphényl]-3'-*C₄*-imidazole-2-carboxamide,
- 9*H*-purin-6-yl-[1,1'-biphényl]-4'-*C₄*-imidazole-2-carboxamide,
- 9*H*-purin-6-yl-[1,1'-biphényl]-3'-*C₄*-imidazole-3-carboxamide,
- 9*H*-purin-6-yl-[1,1'-biphényl]-4'-*C₄*-imidazole-3-carboxamide,
- 9*H*-purin-6-yl-[1,1'-biphényl]-4'-*C₄*-(*N₁*-éthoxyphosphinylméthyl)imidazole-3-carboxamide,
- 7-(phénylméthyl)-7*H*-purin-6-yl-[1,1'-biphényl]-3-carboxamide,
- 9-(phénylméthyl)-9*H*-purin-6-yl-[1,1'-biphényl]-3-carboxamide,
- 7-[(phénylméthoxy)méthyl]-7*H*-purin-6-yl-[1,1'-biphényl]-3-carboxamide,
- 9-[(phénylméthoxy)méthyl]-9*H*-purin-6-yl-[1,1'-biphényl]-3-carboxamide,
- 9-[2-(acétyloxy)éthyl]-9*H*-purin-6-yl-[1,1'-biphényl]-3-carboxamide,
- 9-(2-hydroxyéthyl)-9*H*-purin-6-yl-[1,1'-biphényl]-3-carboxamide,
- 2-[(1,1'-biphényl)-3-carbonylamino-9*H*-purin-6-yl] acétate d'éthyle,
- acide 2-[(1,1'-biphényl)-3-carbonylamino-9*H*-purin-6-yl] acétique,
- [(1,1'-biphényl)-3-carbonylamino-9*H*-purin-6-yl] méthylphosphonate de diéthyle,
- acide [(1,1'-biphényl)-3-carbonylamino-9*H*-purin-6-yl] méthylphosphonique,
- 9*H*-purin-6-yl-naphtalène-1-carboxamide,
- 9*H*-purin-6-yl-naphtalène-2-carboxamide,
- acide (naphtalène-1-carbonylamino-9*H*-purin-6-yl) méthylphosphonique,
- acide (naphtalène-2-carbonylamino-9*H*-purin-6-yl) méthylphosphonique,
- 9*H*-purin-6-yl-[1,1'-biphényl]-4-sulfonamide,
et leurs mélanges.

13. Composition selon la revendication 11 ou la revendication 12 pour son utilisation en tant que médicament.

14. Composition pour une utilisation selon la revendication 13, dans le traitement du cancer.

15. Composition pour une utilisation selon la revendication 13 ou 14, pour une administration simultanée, séparée ou séquentielle dans le traitement du cancer.

16. Composé de formule (I) pour une utilisation selon la revendication 6 ou 7 ou composition pour une utilisation selon la revendication 14 ou 15, dans laquelle le cancer est choisi parmi :
- les tumeurs solides, ou
- les hémopathies aigues, les syndromes myéloprolifératifs chroniques et les syndromes lymphoprolifératifs chroniques, la leucémie lymphoïde chronique, la tricholeucocytose et le myélome multiple.

## Patentansprüche

1. Verbindung mit einem Gerüst des 6-Aminopurin-Typs, **dadurch gekennzeichnet, dass** sie durch die Formel (I) dargestellt ist: 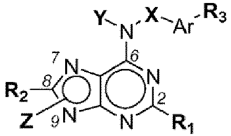 worin,
R₁ und R₂ gleich oder verschieden sind und unabhängig voneinander für Wasserstoff, Alkyl, Alkenyl, Alkinyl, Aryl, Acyl (-COR₉), Amin (-NH₂), Alkylamin (-NHR₉), Dialkylamin (-NR₉R'₉), Acylamin (-NHCOR₉), Diacylamin (-N(COR₉)(COR'₉)), Trifluormethyl (-CF₃), Halogen, Hydroxyl (-OH), Alkoxy (-OR₉), Thio (-SH), Thioalkyl (-SR₉) stehen,
wobei R₉ und R'₉ gleich oder verschieden sind und unabhängig voneinander für Alkyl, Alkenyl, Alkinyl, Aryl stehen,
Z sich an einer der beiden N-7 oder N-9 Positionen des Purins befindet und für Wasserstoff, Alkyl, Alkenyl Alkinyl, Aryl, -COR₉, Halogen, -(CH₂)ₙ-OR₅, -(CH₂)ₙ₁-O-(CH₂)ₙ₂R₅,-(CH₂)_{n'}-COOR₅, -(CH₂)ₙ-P(=O)(OR₆)(OR₇) steht, wobei:
n, n₁ und n₂ gleich oder verschieden sind und unabhängig voneinander für eine ganze Zahl im Bereich von 1 bis 10 stehen und n' für eine ganze Zahl im Bereich von 0 bis 10 steht,
R₅ für Wasserstoff, Alkyl, Alkenyl, Alkinyl, Aryl, -COR₉ steht,
R₆ und R₇ gleich oder verschieden sind und unabhängig voneinander für Wasserstoff, Alkyl, Alkenyl, Alkinyl, Aryl, -COR₉, ein organisches Kation oder ein Metallkation stehen,
X für einen zweiwertigen Rest, ausgewählt aus C=O, C=S, C=NR₈ oder SO₂ steht, wobei:
R₈ für Wasserstoff, Alkyl, Alkenyl, Alkinyl, -OH, -OR₉ steht,
Y die gleiche Bedeutung wie R₅ hat,
Ar für ein Biphenyl steht, das mit einem Substituenten R₃ substituiert sein kann,
R₃ für Wasserstoff, Alkyl, Alkenyl, Alkinyl, Aryl, -NH₂, -NHR₉, -NR₉R'₉, -OH, OR₉, Aryloxy, Benzyloxy (-OCH₂C₆H₅); einen aromatischen oder nicht aromatischen 5- oder 6-gliedrigen Heterozyklus steht, der ein oder mehrere Heteroatome umfasst, ausgewählt aus N, O oder S, wobei der 5- oder 6 gliedrige Heterocyclus noch mit einem Substituenten R₄ substituiert sein kann:
wobei R₄ für Wasserstoff, Alkyl, Alkenyl, Alkinyl, Aryl, -COR₉, -(CH₂)ₙOR₅, -(CH₂)ₙ₁-O-(CH₂)n₂R₅ -(CH₂)_{n'}-COOR₅, -(CH₂)ₙ-P(=O)(OR₆R₇) steht,
wobei R₃ mit Ar in ortho-, meta- oder para-Stellung verknüpft ist,
wobei X mit Ar in ortho-, meta- oder para-Stellung verknüpft ist,
mit Ausnahme der Verbindung 9H-Purin-6-yl-[1,1'-biphenyl]-4-carbonsäureamid.

2. Verbindung gemäß Anspruch 1, **dadurch gekennzeichnet, dass** der Substituent R₃ für Wasserstoff oder einen aromatischen oder nicht aromatischen 5- oder 6-gliedrigen Heterocyclus steht, der ein oder mehrere Heteroatome umfasst, ausgewählt aus N, O oder S, wobei der Heterocyclus mit einem wie in Anspruch 1 definierten Substituenten R₄ substituiert sein kann.

3. Verbindung gemäß Anspruch 1 oder 2, **dadurch gekennzeichnet, dass** der 5-gliedrige Heterocyclus für ein Pyrrol oder ein Imidazol steht, wobei das Pyrrol oder das Imidazol mit einem Substituenten R₄, der ausgewählt ist aus Wasserstoff oder -(CH₂)ₙ-P(=O)(OR₆)(OR₇), wie in Anspruch 1 definiert, substituiert sein kann.

4. Verbindung gemäß einem der Ansprüche 1 bis 3, **dadurch gekennzeichnet, dass**:
R₁ für Wasserstoff, (-NH₂), (-NHR₉), (-NR₉R'₉), (-NHCOR₉), (-N(COR₉)(COR'₉)) steht,
R₂ für Wasserstoff, Benzyl, Phenyl steht,
Z für Wasserstoff, Benzyl, Phenyl, -(CH₂)ₙ-OR₅, -(CH₂)ₙ₁-O-(CH₂)ₙ₂R₅, -(CH₂)_{n'}-COOR₅, -(CH₂)ₙ-P(=O)(OR₆)(OR₇) steht,
wobei: n, n₁, n₂ und n' gleich oder verschieden sind und unabhängig voneinander für eine ganze Zahl gleich 1 oder 2 stehen,
R₅ für Wasserstoff, Ethyl, Acetyl (-COCH₃), Phenyl, stehen,
R₆ und R₇ gleich oder verschieden sind und unabhängig voneinander für Wasserstoff, Methyl, Ethyl, ein Natriumkation (Na⁺), ein Lithiumkation (Li⁺) stehen,
X für C=O, SO₂ steht,
Y für Wasserstoff steht.

5. Verbindung gemäß einem der Ansprüche 1 bis 4, **dadurch gekennzeichnet, dass** sie ausgewählt ist aus der Gruppe umfassend:
- 9*H*-Purin-6-yl-[1,1'-biphenyl]-2-carbonsäureamid,
- 9*H*-Purin-6-yl-[1,1'-biphenyl]-3-carbonsäureamid,
- 9*H*-Purin-6-yl-[1,1'-biphenyl]-3'-*N*-pyrrol-2-carbonsäureamid,
- 9*H*-Purin-6-yl-[1,1'-biphenyl]-4'-*N*-pyrrol-2-carbonsäureamid,
- 9*H*-Purin-6-yl-[1,1'-biphenyl]-3'-*N*-pyrrol-3-carbonsäureamid,
- 9*H*-Purin-6-yl-[1,1'-biphenyl]-4'-*N*-pyrrol-3-carbonsäureamid,
- 9*H*-Purin-6-yl-[1,1'-biphenyl]-3'-*N*-pyrrol-4-carbonsäureamid,
- 9*H*-Purin-6-yl-[1,1'-biphenyl]-4'-*N*-Pyrrol-4-carbonsäureamid,
- 9*H*-Purin-6-yl-[1,1'-biphenyl]-3'-*N*-imidazol-2-carbonsäureamid,
- 9*H*-Purin-6-yl-[1,1'-biphenyl]-4'-*N*-imidazol-2-carbonsäureamid,
- 9*H*-Purin-6-yl-[1,1'-biphenyl]-3'-*N*-imidazol-3-carbonsäureamid,
- 9*H*-Purin-6-yl-[1,1'-biphenyl]-4'-*N*-imidazol-3-carbonsäureamid,
- 9*H*-Purin-6-yl-[1,1'-biphenyl]-3'-*C₄*-imidazol-2-carbonsäureamid,
- 9*H*-Purin-6-yl-[1,1'-biphenyl]-4'-*C₄*-imidazol-2-carbonsäureamid,
- 9*H*-Purin-6-yl-[1,1'-bipheny1]-3'-*C₄*-imidazol-3-carbonsäureamid,
- 9*H*-Purin-6-yl-[1,1'-bipheny11-4'-*C₄*-imidazol-3-carbonsäureamid,
- 9*H*-Purin-6-yl-[1,1'-biphenyl]-4'-*C₄*-(*N₁*-ethoxyphosphinylmethyl)imidazol-3-carbonsäureamid,
- 7-(Phenylmethyl)-7*H*-purin-6-yl-[1,1'-biphenyl]-3-carbonsäureamid,
- 9-(Phenylmethyl)-9*H*-purin-6-yl-[1,1'-biphenyl]-3-carbonsäureamid,
- 7-[(Phenylmethoxy)methyl]-7*H*-purin-6-yl-[1,1'-biphenyl]-3-carbonsäureamid,
- 9-[(Phenylmethoxy)methyl]-9*H*-purin-6-yl-[1,1'-biphenyl]-3-carbonsäureamid,
- 9-[2-(Acetyloxy)ethyl]-9*H*-purin-6-yl-[1,1'-biphenyl]-3-carbonsäureamid,
- 9-(2-Hydroxyethyl)-9*H*-purin-6-yl-[1,1'-biphenyl]-3-carbonsäureamid,
- 2-[(1,1'-biphenyl)-3-carbonylamino-9*H*-purin-6-yl]ethylacetat
- 2-[(1,1'-biphenyl)-3-carbonylamino-9*H*-purin-6-yl]essigsäure,
- [(1,1'-biphenyl)-3-carbonylamino-9*H*-purin-6-yl]diethylmethylphosphonat,
- [(1,1'-biphenyl)-3-carbonylamino-9*H*-purin-6-yl]methylphosphonsäure und Mischungen davon.

6. Verbindung mit einem Gerüst des 6-Aminopurin-Typs, **dadurch gekennzeichnet, dass** sie durch die Formel (I) dargestellt ist: 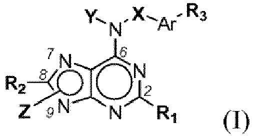 worin
R₁, R₂, R₃, X, Y und Z wie in einem der Ansprüche 1 bis 5 definiert sind,
Ar für ein Biphenyl oder ein Naphthyl steht, wobei das Biphenyl oder das Naphthyl mit einem Substituenten R₃ substituiert sein kann,
wobei R₃ mit Ar in ortho-, meta- oder para-Stellung verknüpft ist,
wobei X mit Ar in ortho-, meta- oder para-Stellung verknüpft ist,
wobei die Verbindung für die Verwendung in der Behandlung von Krebs bestimmt ist.

7. Verbindung der Formel (I) für die Verwendung gemäß Anspruch 6, **dadurch gekennzeichnet, dass** sie ausgewählt ist aus der Gruppe umfassend:
- 9*H*-Purin-6-yl-[1,1'-biphenyl]-2-carbonsäureamid,
- 9*H*-Purin-6-yl-[1,1'-biphenyl]-3-carbonsäureamid,
- 9*H*-Purin-6-yl-[1,1'-biphenyl]-4-carbonsäureamid,
- 9*H*-Purin-6-yl-[1,1'-biphenyl]-3'-*N*-pyrrol-2-carbonsäureamid,
- 9*H*-Purin-6-yl-[1,1'-biphenyl]-4'-*N*-pyrrol-2-carbonsäureamid,
- 9*H*-Purin-6-yl-[1,1'-biphenyl]-3'-*N*-pyrrol-3-carbonsäureamid,
- 9*H*-Purin-6-yl-[1,1'-biphenyl]-4'-*N*-pyrrol-3-carbonsäureamid,
- 9*H*-Purin-6-yl-[1,1'-biphenyl]-3'-*N*-pyrrol-4-carbonsäureamid,
- 9*H*-Purin-6-yl-[1,1'-biphenyl]-4'-*N*-Pyrrol-4-carbonsäureamid,
- 9*H*-Purin-6-yl-[1,1'-biphenyl]-3'-*N*-imidazol-2-carbonsäureamid,
- 9*H*-Purin-6-yl-[1,1'-biphenyl]-4'-*N*-imidazol-2-carbonsäureamid,
- 9*H*-Purin-6-yl-[1,1'-biphenyl]-3'-*N*-imidazol-3-carbonsäureamid,
- 9*H*-Purin-6-yl-[1,1'-biphenyl]-4'-*N*-imidazol-3-carbonsäureamid,
- 9*H*-Purin-6-yl-[1,1'-biphenyl]-3'-*C₄*-imidazol-2-carbonsäureamid,
- 9*H*-Purin-6-yl-[1,1'-biphenyl]-4'-*C₄*-imidazol-2-carbonsäureamid,
- 9*H*-Purin-6-yl-[1,1'-biphenyl]-3'-*C₄*-imidazol-3-carbonsäureamid,
- 9*H*-Purin-6-yl-[1,1'-biphenyl]-4'-*C₄*-imidazol-3-carbonsäureamid,
- 9*H*-Purin-6-yl-[1,1'-biphenyl]-4'-*C₄*-(*N₁*-ethoxyphosphinylmethyl)imidazol-3-carbonsäureamid,
- 7-(Phenylmethyl)-7*H*-purin-6-yl-[1,1'-biphenyl]-3-carbonsäureamid,
- 9-(Phenylmethyl)-9*H*-purin-6-yl-[1,1'-biphenyl]-3-carbonsäureamid,
- 7-[(Phenylmethoxy)methyl]-7*H*-purin-6-yl-[1,1'-biphenyl]-3-carbonsäureamid,
- 9-[(Phenylmethoxy)methyl]-9*H*-purin-6-yl-[1,1'-biphenyl]-3-carbonsäureamid,
- 9-[2-(Acetyloxy)ethyl]-9*H*-purin-6-yl-[1,1'-biphenyl]-3-carbonsäureamid,
- 9-(2-Hydroxyethyl)-9*H*-purin-6-yl-[1,1'-biphenyl]-3-carbonsäureamid,
- 2-[(1,1'-biphenyl)-3-carbonylamino-9H-purin-6-yl]ethylacetat
- 2-[(1,1'-biphenyl)-3-carbonylamino-9*H*-purin-6-yl]essigsäure,
- [(1,1'-biphenyl)-3-carbonylamino-9*H*-purin-6-yl]diethylmethylphosphonat,
- [(1,1'-biphenyl)-3-carbonylamino-9*H*-purin-6-yl]methylphosphonsäure,
- 9*H*-Purin-6-yl-naphthyl-1-carbonsäureamid,
- 9*H*-Purin-6-yl-naphthyl-2-carbonsäureamid,
- (Naphthyl-1-carbonylamino-9*H*-purin-6-yl)methylphosphonsäure,
- (Naphthyl-2-carbonylamino-9H-purin-6-yl)methylphosphonsäure,
- 9*H*-purin-6-yl-[1,1'-Biphenyl]-4-sulfonamid,
und Mischungen davon.

8. Verbindung der Formel (I) für die Verwendung gemäß Anspruch 6 oder 7, zur Hemmung von mindestens einer 5'-Nukleotidase, die ausgewählt ist aus zytosolischer 5'-Nukleotidase II (cN-II), zytosolischer 5'-Nukleotidase IA (cN-IA), zytosolischer 5'-Nukleotidase IB (cN-IB), zytosolischer 5'-Nukleotidase IIIA (cN-IIIA), zytosolischer 5'-Nukleotidase IIIB (cN-IIIB), Ecto-5'-Nucleotidase (eN,CD73), zytosolischer 5'(3')-Desoxynukleotidase (cdN) oder mitochondrialer 5'(3')-Desoxynukleotidase (mdN).

9. Verbindung der Formel (I) für die Verwendung gemäß einem der Ansprüche 6 bis 8, **dadurch gekennzeichnet, dass** sie mit mindestens einem Nukleosid-Analogon und/oder mindestens einem Nukleobase-Analogon verbunden ist, um die Antikrebswirkung des Nukleosid-Analogons und/oder des Nukleobase-Analogons zu verstärken.

10. Verbindung der Formel (I) für die Verwendung gemäß Anspruch 9, wobei:
- das Nukleosid-Analogon ausgewählt ist aus Cladribin, Fludarabin, Clofarabin, Cytarabin, Gemcitabin, Nelarabin, Floxuridin oder Pentostatin,
- das Nukleobase-Analogon ausgewählt ist aus Fluorouracil, 6-Mercaptopurin oder 6-Thioguanosin.

11. Zusammensetzung, umfassend:
- mindestens eine Verbindung der Formel (I): 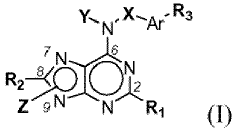 worin
R₁, R₂, R₃, X, Y und Z wie in einem der Ansprüche 1 bis 5 definiert sind,
Ar für ein Biphenyl oder ein Naphthyl steht, wobei das Biphenyl oder das Naphthyl mit einem Substituenten R₃ substituiert sein kann,
wobei R₃ mit Ar in ortho-, meta- oder para-Stellung verknüpft ist,
wobei X mit Ar in ortho-, meta- oder para-Stellung verknüpft ist,
in Verbindung mit:
- mindestens einem Nukleosid-Analogon, ausgewählt aus Cladribin, Fludarabin, Clofarabin, Cytarabin, Gemcitabin, Nelarabin, Floxuridin oder Pentostatin, und/oder
- mindestens einem Nukleobase-Analogon, ausgewählt aus Fluorouracil, 6-Mercaptopurin oder 6-Thioguanosin.
- und gegebenenfalls mindestens einem pharmazeutisch verträglichen Hilfsstoff.

12. Zusammensetzung gemäß Anspruch 11, wobei die Verbindung der Formel (I) ausgewählt ist aus der Gruppe umfassend:
- 9*H*-Purin-6-yl-[1,1'-biphenyl]-2-carbonsäureamid,
- 9*H*-Purin-6-yl-[1,1'-biphenyl]-3-carbonsäureamid,
- 9*H*-Purin-6-yl-[1,1'-biphenyl]-4-carbonsäureamid,
- 9*H*-Purin-6-yl-[1,1'-biphenyl]-3'-*N*-pyrrol-2-carbonsäureamid,
- 9*H*-Purin-6-yl-[1,1'-biphenyl]-4'-*N*-pyrrol-2-carbonsäureamid,
- 9*H*-Purin-6-yl-[1,1'-biphenyl]-3'-*N*-pyrrol-3-carbonsäureamid,
- 9*H*-Purin-6-yl-[1,1'-biphenyl]-4'-*N*-pyrrol-3-carbonsäureamid,
- 9*H*-Purin-6-yl-[1,1'-biphenyl]-3'-*N*-pyrrol-4-carbonsäureamid,
- 9*H*-Purin-6-yl-[1,1'-biphenyl]-4'-*N*-Pyrrol-4-carbonsäureamid,
- 9*H*-Purin-6-yl-[1,1'-biphenyl]-3'-*N*-imidazol-2-carbonsäureamid,
- 9*H*-Purin-6-yl-[1,1'-biphenyl]-4'-*N*-imidazol-2-carbonsäureamid,
- 9*H*-Purin-6-yl-[1,1'-biphenyl]-3'-*N*-imidazol-3-carbonsäureamid,
- 9*H*-Purin-6-yl-[1,1'-biphenyl]-4'-*N*-imidazol-3-carbonsäureamid,
- 9*H*-Purin-6-yl-[1,1'-biphenyl]-3'-*C₄*-imidazol-2-carbonsäureamid,
- 9*H*-Purin-6-yl-[1,1'-biphenyl]-4'-*C₄*-imidazol-2-carbonsäureamid,
- 9*H*-Purin-6-yl-[1,1'-biphenyl]-3'-*C₄*-imidazol-3-carbonsäureamid,
- 9*H*-Purin-6-yl-[1,1'-biphenyl]-4'-*C₄*-imidazol-3-carbonsäureamid,
- 9*H*-Purin-6-yl-[1,1'-biphenyl]-4'-*C₄*-(*N₁*-ethoxyphosphinylmethyl)imidazol-3-carbonsäureamid,
- 7-(Phenylmethyl)-7*H*-purin-6-yl-[1,1'-biphenyl]-3-carbonsäureamid,
- 9-(Phenylmethyl)-9*H*-purin-6-yl-[1,1'-biphenyl]-3-carbonsäureamid,
- 7-[(Phenylmethoxy)methyl]-7*H*-purin-6-yl-[1,1'-biphenyl]-3-carbonsäureamid,
- 9-[(Phenylmethoxy)methyl]-9*H*-purin-6-yl-[1,1'-biphenyl]-3-carbonsäureamid,
- 9-[2-(Acetyloxy)ethyl]-9*H*-purin-6-yl-[1,1'-biphenyl]-3-carbonsäureamid,
- 9-(2-Hydroxyethyl)-9*H*-purin-6-yl-[1,1'-biphenyl]-3-carbonsäureamid,
- 2-[(1,1'-Biphenyl)-3-carbonylamino-9*H*-purin-6-yl]ethylacetat
- 2-[(1,1'-Biphenyl)-3-carbonylamino-9H-purin-6-yl]essigsäure,
- [(1,1'-Biphenyl)-3-carbonylamino-9*H*-purin-6-yl]diethylmethylphosphonat,
- [(1,1'-Biphenyl)-3-carbonylamino-9*H*-purin-6-yl]methylphosphonsäure,
- 9*H*-Purin-6-yl-naphthyl-1-carbonsäureamid,
- 9*H*-Purin-6-yl-naphthyl-2-carbonsäureamid,
- (Naphthyl-1-carbonylamino-9*H*-purin-6-yl)methylphosphonsäure,
- (Naphthyl-2-carbonylamino-9*H*-purin-6-yl)methylphosphonsäure,
- 9*H*-Purin-6-yl-[1,1'-biphenyl]-4-sulfonamid,
und Mischungen davon.

13. Zusammensetzung gemäß Anspruch 11 oder Anspruch 12 für die Verwendung als Medikament.

14. Zusammensetzung für eine Verwendung gemäß Anspruch 13, zur Behandlung von Krebs.

15. Zusammensetzung für eine Verwendung gemäß Anspruch 13 oder 14, zur gleichzeitigen, getrennten oder aufeinanderfolgenden Verabreichung bei der Behandlung von Krebs.

16. Verbindung der Formel (I) für die Verwendung gemäß Anspruch 6 oder 7 oder Zusammensetzung für die Verwendung gemäß Anspruch 14 oder 15, wobei der Krebs ausgewählt ist aus :
- soliden Tumoren, oder
- akuten malignen hämatologischen Erkrankungen, chronischen myeloproliferativen Syndromen und chronischen lymphoproliferativen Syndromen, chronischer lymphatischer Leukämie, Haarzellleukämie und Multiplem Myelom.

## Claims

1. A compound which has a 6-aminopurine backbone, **characterized in that** it has the formula: 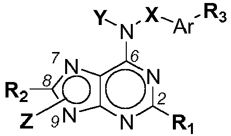 wherein
R₁ and R₂, which may be identical or different, represent, independently of one another, hydrogen, alkyl, alkenyl, alkynyl, aryl, acyl (-COR₉), amino (-NH₂), alkylamino (-NHR₉), dialkylamino (-NR₉R'₉), acylamino (-NHCOR₉), diacylamino (-N(COR₉)(COR'₉)), trifluoromethyl (-CF₃), halogen, hydroxyl (-OH), alkoxy (-OR₉), thio (-SH) or thioalkyl (-SR₉),
with R₉ and R'₉, which may be identical or different, representing, independently of one another, alkyl, alkenyl, alkynyl or aryl,
Z is on either of the positions N₇ and N₉ of the purine, and represents hydrogen, alkyl, alkenyl, alkynyl, aryl, -COR₉, halogen, -(CH₂)ₙ-OR₅, -(CH₂)ₙ₁-O-(CH₂)ₙ₂R₅, -(CH₂)_{n'}-COOR₅ or -(CH₂)ₙ-P(=O)(OR₆)(OR₇), with:
n, n₁ and n₂, which may be identical or different, representing, independently of one another, an integer ranging from 1 to 10 and n' an integer ranging from 0 to 10,
R₅ representing hydrogen, alkyl, alkenyl, alkynyl, aryl or -COR₉,
R₆ and R₇, which may be identical or different, representing, independently of one another, hydrogen, alkyl, alkenyl, alkynyl, aryl, -COR₉, an organic cation or a metal cation,
X representi a divalent radical chosen from C=O, C=S, C=NR₈ or SO₂, with:
R₈ representing hydrogen, alkyl, alkenyl, alkynyl, -OH or -OR₉,
Y has the same meaning as R₅,
Ar represents a biphenyl which may be substituted with a substituent R₃,
R₃ represents hydrogen, alkyl, alkenyl, alkynyl, aryl, -NH₂, -NHR₉, -NR₉R'₉, -OH, -OR₉, aryloxy, benzyloxy (-OCH₂C₆H₅); a 5-membered or 6-membered aromatic or nonaromatic heterocycle comprising one or more heteroatoms chosen from N, O or S, said 5-membered or 6-membered heterocycle also possibly being substituted with a substituent R₄, with:
R₄ representing hydrogen, alkyl, alkenyl, alkynyl, aryl, -COR₉, -(CH₂)ₙ₋OR₅, -(CH₂)ₙ₁-O-(CH₂)ₙ₂R₅, -(CH₂)_{n'}-COOR₅ or -(CH₂)ₙ-P(=O)(OR₆R₇),
R₃ being bonded to Ar in the ortho, meta or para position,
X being bonded to Ar in the ortho, meta or para position,
with the exception of the compound 9*H*-purin-6-yl-[1,1'-biphenyl]-4-carboxamide.

2. The compound as claimed in claim 1, **characterized in that** the substituent R₃ represents a hydrogen or a 5-membered or 6-membered aromatic or nonaromatic heterocycle comprising one or more heteroatoms chosen from N, O or S, said heterocycle possibly being substituted with a substituent R₄ as defined in claim 1.

3. The compound as claimed in claim 1 or 2, **characterized in that** the 5-membered heterocycle represents a pyrrole or an imidazole, said pyrrole or imidazole possibly being substituted with a substituent R₄ chosen from hydrogen or -(CH₂)ₙ-P(=O)(OR₆)(OR₇) as defined in claim 1.

4. The compound as claimed in any one of claims 1 to 3, **characterized in that**:
R₁ represents hydrogen, (-NH₂), (-NHR₉), (-NR₉R'₉), (-NHCOR₉), (-N(COR₉)(COR'₉)),
R₂ represents hydrogen, benzyl or phenyl,
Z represents hydrogen, benzyl, phenyl, -(CH₂)ₙ-OR₅, -(CH₂)ₙ₁-O-(CH₂)ₙ₂R₅, -(CH₂)_{n'}-COOR₅ or -(CH₂)ₙ-P(=O)(OR₆)(OR₇) with:
n, n₁, n₂ and n', which may be identical or different, representing, independently of one another, an integer equal to 1 or 2,
R₅ representing hydrogen, ethyl, acetyl (-COCH₃) or phenyl,
R₆ and R₇, which may be identical or different, representing, independently of one another, hydrogen, methyl, ethyl, a sodium cation (Na⁺) or a lithium cation (Li⁺),
X represents C=O, SO₂,
Y represents hydrogen.

5. The compound as claimed in any one of claims 1 to 4, **characterized in that** it is chosen from the group comprising:
- 9*H*-purin-6-yl-[1,1'-biphenyl]-2-carboxamide,
- 9*H*-purin-6-yl-[1,1'-biphenyl]-3-carboxamide,
- 9*H*-purin-6-yl-[1,1'-biphenyl]-3'-*N-*pyrrole-2-carboxamide,
- 9*H*-purin-6-yl-[1,1'-biphenyl]-4'-*N*-pyrrole-2-carboxamide,
- 9*H*-purin-6-yl-[1,1'-biphenyl]-3 '-*N*-pyrrole-3-carboxamide,
- 9*H*-purin-6-yl-[1,1'-biphenyl]-4'-*N*-pyrrole-3-carboxamide,
- 9*H*-purin-6-yl-[1,1'-biphenyl]-3 '-*N*-pyrrole-4-carboxamide,
- 9*H*-purin-6-yl-[1,1'-biphenyl]-4'-*N*-pyrrole-4-carboxamide,
- 9*H*-purin-6-yl-[1,1'-biphenyl]-3 '-*N*-imidazole-2-carboxamide,
- 9*H*-purin-6-yl-[1,1'-biphenyl]-4'-*N*-imidazole-2-carboxamide,
- 9*H*-purin-6-yl-[1,1'-biphenyl]-3'-*N*-imidazole-3-carboxamide,
- 9*H*-purin-6-yl-[1,1'-biphenyl]-4'-*N*-imidazole-3-carboxamide,
- 9*H*-purin-6-yl-[1,1'-biphenyl]-3'-*C₄*-imidazole-2-carboxamide,
- 9*H*-purin-6-yl-[1,1'-biphenyl]-4'-*C₄*-imidazole-2-carboxamide,
- 9*H*-purin-6-yl-[1,1'-biphenyl]-3'-*C₄*-imidazole-3-carboxamide,
- 9*H*-purin-6-yl-[1,1'-biphenyl]-4'-*C₄*-imidazole-3-carboxamide,
- 9*H*-purin-6-yl-[1,1'-biphenyl]-4'-*C₄*-(*N₁*-ethoxyphosphinylmethyl)imidazole-3-carboxamide,
- 7-(phenylmethyl)-7*H*-purin-6-yl-[1,1'-biphenyl]-3-carboxamide,
- 9-(phenylmethyl)-9*H*-purin-6-yl-[1,1'-biphenyl]-3-carboxamide,
- 7-[(phenylmethoxy)methyl]-7*H*-purin-6-yl-[1,1'-biphenyl]-3-carboxamide,
- 9-[(phenylmethoxy)methyl]-9*H*-purin-6-yl-[1,1'-biphenyl]-3-carboxamide,
- 9-[2-(acetyloxy)ethyl]-9*H*-purin-6-yl-[1,1'-biphenyl]-3-carboxamide,
- 9-(2-hydroxyethyl)-9*H*-purin-6-yl-[1,1'-biphenyl]-3-carboxamide,
- ethyl 2-[(1,1'-biphenyl)-3-carbonylamino-9*H*-purin-6-yl]acetate,
- 2-[(1,1'-biphenyl)-3-carbonylamino-9*H*-purin-6-yl]acetic acid,
- diethyl [(1,1'-biphenyl)-3-carbonylamino-9*H-*purin-6-yl]methylphosphonate,
- [(1,1'-biphenyl)-3-carbonylamino-9*H*-purin-6-yl]methylphosphonic acid,
and mixtures thereof.

6. A compound which has a 6-aminopurine backbone, **characterized in that** it has formula (I): 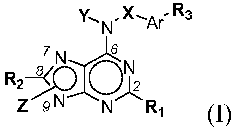 wherein
R₁, R₂, R₃, X, Y and Z are as defined in any one of claims 1 to 5,
Ar represents a biphenyl or a naphthyl, said biphenyl or naphthyl possibly being substituted with a substituent R₃,
R₃ being bonded to Ar in the ortho, meta or para position,
X being bonded to Ar in the ortho, meta or para position,
said compound being intended for use in the treatment of cancer.

7. The compound of formula (I) for use as claimed in claim 6, **characterized in that** it is chosen from the group comprising:
- 9*H*-purin-6-yl-[1,1'-biphenyl]-2-carboxamide,
- 9*H*-purin-6-yl-[1,1'-biphenyl]-3-carboxamide,
- 9*H*-purin-6-yl-[1,1'-biphenyl]-4-carboxamide,
- 9*H*-purin-6-yl-[1,1'-biphenyl]-3'-*N*-pyrrole-2-carboxamide,
- 9*H*-purin-6-yl-[1,1'-biphenyl]-4'-*N*-pyrrole-2-carboxamide,
- 9*H*-purin-6-yl-[1,1'-biphenyl]-3'-*N*-pyrrole-3-carboxamide,
- 9*H*-purin-6-yl-[1,1'-biphenyl]-4'-*N*-pyrrole-3-carboxamide,
- 9*H*-purin-6-yl-[1,1'-biphenyl]-3'-*N*-pyrrole-4-carboxamide,
- 9*H*-purin-6-yl-[1,1'-biphenyl]-4'-*N*-pyrrole-4-carboxamide,
- 9*H*-purin-6-yl-[1,1'-biphenyl]-3'-*N*-imidazole-2-carboxamide,
- 9*H*-purin-6-yl-[1,1'-biphenyl]-4'-*N*-imidazole-2-carboxamide,
- 9*H*-purin-6-yl-[1,1'-biphenyl]-3'-*N*-imidazole-3-carboxamide,
- 9*H*-purin-6-yl-[1,1'-biphenyl]-4'-*N*-imidazole-3-carboxamide,
- 9*H*-purin-6-yl-[1,1'-biphenyl]-3'-*C₄*-imidazole-2-carboxamide,
- 9*H*-purin-6-yl-[1,1'-biphenyl]-4'-*C₄*-imidazole-2-carboxamide,
- 9*H*-purin-6-yl-[1,1'-biphenyl]-3'-*C₄*-imidazole-3-carboxamide,
- 9*H*-purin-6-yl-[1,1'-biphenyl]-4'-*C₄*-imidazole-3-carboxamide,
- 9*H-*purin-6-yl-[1,1'-biphenyl]-4'-*C*_{*4*-}(*N₁*-ethoxyphosphinylmethyl)imidazole-3-carboxamide,
- 7-(phenylmethyl)-7*H-*purin-6-yl-[1,1'-biphenyl]-3-carboxamide,
- 9-(phenylmethyl)-9*H*-purin-6-yl-[1,1'-biphenyl]-3-carboxamide,
- 7-[(phenylmethoxy)methyl]-7*H*-purin-6-yl-[1,1'-biphenyl]-3-carboxamide,
- 9-[(phenylmethoxy)methyl]-9*H*-purin-6-yl-[1,1'-biphenyl]-3-carboxamide,
- 9-[2-(acetyloxy)ethyl]-9*H*-purin-6-yl-[1,1'-biphenyl]-3-carboxamide,
- 9-(2-hydroxyethyl)-9*H*-purin-6-yl-[1,1'-biphenyl]-3-carboxamide,
- ethyl 2-[(1,1'-biphenyl)-3-carbonylamino-9*H-*purin-6-yl]acetate,
- 2-[(1,1'-biphenyl)-3-carbonylamino-9*H*-purin-6-yl]acetic acid,
- diethyl [(1,1'-biphenyl)-3-carbonylamino-9*H-*purin-6-yl]methylphosphonate,
- [(1,1'-biphenyl)-3-carbonylamino-9*H*-purin-6-yl]methylphosphonic acid,
- 9*H*-purin-6-ylnaphthalene-1-carboxamide,
- 9*H*-purin-6-ylnaphthalene-2-carboxamide,
- (naphthalene-1-carbonylamino-9*H-*purin-6-yl)methylphosphonic acid,
- (naphthalene-2-carbonylamino-9*H*-purin-6-yl)methylphosphonic acid,
- 9*H*-purin-6-yl-[1,1'-biphenyl]-4-sulfonamide,
and mixtures thereof.

8. The compound of formula (I) for use as claimed in claim 6 or 7, for inhibiting at least one 5'-nucleotidase chosen from cytosolic 5'-nucleotidase II (cN-II), cytosolic 5'-nucleotidase IA (cN-IA), cytosolic 5'-nucleotidase IB (cN-IB), cytosolic 5'-nucleotidase IIIA (cN-IIIA), cytosolic 5'-nucleotidase IIIB (cN-IIIB), ecto-5'-nucleotidase (eN, CD73), cytosolic 5'(3')-deoxynucleotidase (cdN) or mitochondrial 5'(3')-deoxynucleotidase (mdN).

9. The compound of formula (I) for use as claimed in any one of claims 6 to 8, **characterized in that** is combined with at least one nucleoside analog and/or at least one nucleobase analog for potentiating the anticancer effect of said nucleoside analog and/or nucleobase analog.

10. The compound of formula (I) for use as claimed in claim 9, wherein:
- the nucleoside analog is chosen from cladribine, fludarabine, clofarabine, cytarabine, gemcitabine, nelarabine, floxuridine or pentostatin,
- the nucleobase analog is chosen from fluorouracil, 6-mercaptopurine or 6-thioguanosine.

11. A composition comprising:
- at least one compound of formula (I): 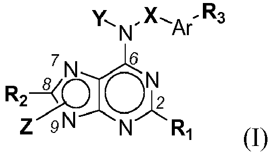 wherein
R₁, R₂, R₃, X, Y and Z are as defined in any one of claims 1 to 5,
Ar represents a biphenyl or a naphthyl, said biphenyl or naphthyl possibly being substituted with a substituent R₃,
R₃ being bonded to Ar in the ortho, meta or para position,
X being bonded to Ar in the ortho, meta or para position,
in combination with:
- at least one nucleoside analog chosen from cladribine, fludarabine, clofarabine cytarabine, gemcitabine, nelarabine, floxuridine or pentostatin, and/or
- at least one nucleobase analog chosen from fluorouracil, 6-mercaptopurine or 6-thioguanosine,
- and optionally at least one pharmaceutically acceptable excipient.

12. The composition as claimed in claim 11, wherein the compound of formula (I) is chosen from the group comprising:
- 9*H*-purin-6-yl-[1,1'-biphenyl]-2-carboxamide,
- 9*H*-purin-6-yl-[1,1'-biphenyl]-3-carboxamide,
- 9*H*-purin-6-yl-[1,1'-biphenyl]-4-carboxamide,
- 9*H*-purin-6-yl-[1,1'-biphenyl]-3'-*N*-pyrrole-2-carboxamide,
- 9*H*-purin-6-yl-[1,1'-biphenyl]-4'-*N*-pyrrole-2-carboxamide,
- 9*H*-purin-6-yl-[1,1'-biphenyl]-3 '-*N*-pyrrole-3-carboxamide,
- 9*H*-purin-6-yl-[1,1'-biphenyl]-4'-*N*-pyrrole-3-carboxamide,
- 9*H*-purin-6-yl-[1,1'-biphenyl]-3'-*N*-pyrrole-4-carboxamide,
- 9*H*-purin-6-yl-[1,1'-biphenyl]-4'-*N*-pyrrole-4-carboxamide,
- 9*H*-purin-6-yl-[1,1'-biphenyl]-3 '-*N-*imidazole-2-carboxamide,
- 9*H*-purin-6-yl-[1,1'-biphenyl]-4'-*N*-imidazole-2-carboxamide,
- 9*H*-purin-6-yl-[1,1'-biphenyl]-3 '-*N-*imidazole-3-carboxamide,
- 9*H*-purin-6-yl-[1,1'-biphenyl]-4'-*N*-imidazole-3-carboxamide,
- 9*H*-purin-6-yl-[1,1'-biphenyl]-3'-*C₄*-imidazole-2-carboxamide,
- 9*H*-purin-6-yl-[1,1'-biphenyl]-4'-*C₄*-imidazole-2-carboxamide,
- 9*H*-purin-6-yl-[1,1'-biphenyl]-3'-*C₄*-imidazole-3-carboxamide,
- 9*H*-purin-6-yl-[1,1'-biphenyl]-4'-*C₄*-imidazole-3-carboxamide,
- 9*H*-purin-6-yl-[1,1'-biphenyl]-4'-*C₄*-(*N₁*-ethoxyphosphinylmethyl)imidazole-3-carboxamide,
- 7-(phenylmethyl)-7*H*-purin-6-yl-[1,1'-biphenyl]-3-carboxamide,
- 9-(phenylmethyl)-9*H*-purin-6-yl-[1,1'-biphenyl]-3-carboxamide,
- 7-[(phenylmethoxy)methyl]-7*H*-purin-6-yl-[1,1'-biphenyl]-3-carboxamide,
- 9-[(phenylmethoxy)methyl]-9*H*-purin-6-yl-[1,1'-biphenyl]-3-carboxamide,
- 9-[2-(acetyloxy)ethyl]-9*H*-purin-6-yl-[1,1'-biphenyl]-3-carboxamide,
- 9-(2-hydroxyethyl)-9*H*-purin-6-yl-[1,1'-biphenyl]-3-carboxamide,
- ethyl 2-[(1,1'-biphenyl)-3-carbonylamino-9*H-*purin-6-yl]acetate,
- 2-[(1,1'-biphenyl)-3-carbonylamino-9*H*-purin-6-yl]acetic acid,
- diethyl [(1,1'-biphenyl)-3-carbonylamino-9*H*-purin-6-yl]methylphosphonate,
- [(1,1'-biphenyl)-3-carbonylamino-9*H*-purin-6-yl]methylphosphonic acid,
- 9*H*-purin-6-ylnaphthalene-1-carboxamide,
- 9*H*-purin-6-ylnaphthalene-2-carboxamide,
- (naphthalene-1-carbonylamino-9*H-*purin-6-yl)methylphosphonic acid,
- (naphthalene-2-carbonylamino-9*H*-purin-6-yl)methylphosphonic acid,
- 9*H*-purin-6-yl-[1,1'-biphenyl]-4-sulfonamide,
and mixtures thereof.

13. The composition as claimed in claim 11 or claim 12, for use thereof as a medicament.

14. The composition for use as claimed in claim 13, in the treatment of cancer.

15. The composition for use as claimed in claim 13 or 14, for simultaneous, separate or sequential administration in the treatment of cancer.

16. The compound of formula (I) for use as claimed in claim 6 or 7 or the composition for use as claimed in claim 14 or 15, wherein the cancer is chosen from:
- solid tumors, or
- acute hemopathies, chronic myeloproliferative syndromes and chronic lymphoproliferative syndromes, chronic lymphoid leukemia, hairy cell lymphoma and multiple myeloma.
